# EUROPÄISCHE PATENTSCHRIFT

(11) **EP 1 742 058 B1**
(45) Veröffentlichungstag und Bekanntmachung des Hinweises auf die Patenterteilung: **30.11.2011**
(21) Anmeldenummer: 06020037.5
(22) Anmeldetag: 27.08.1999
(51) Int. Cl.: G01N 33/551, G01N 33/543, B01J 19/00, B01L 3/00

(54) **Träger für Analytbestimmungsverfahren und Verfahren zur Herstellung des Trägers**
Substrate for analyte determination and methods for manufacturing such substrates
Substrats pour procédés de détermination d'analytes et méthodes de fabrication de tels substrats

(30) Priorität: 28.08.1998 DE 19839256; 28.08.1998 DE 19839254; 28.08.1998 DE 19839255; 19.02.1999 DE 19907080; 27.05.1999 DE 19924327
(43) Veröffentlichungstag der Anmeldung: 10.01.2007
(62) Teilanmeldung aus: 03028140.6
(73) Patentinhaber: febit holding GmbH, 69120 Heidelberg (DE)
(72) Erfinder: Stähler, Cord F., 69469 Weinheim (DE); Stähler, Peer F., 68167 Mannheim (DE); Müller, Manfred, 80689 München (DE); Stähler, Fritz, Dr., 69469 Weinheim (DE); Lindner, Hans, 70569 Stuttgart (DE)
(74) Vertreter: Zwicker, Jörk

(56) Entgegenhaltungen:
- WO-A-98/13683
- US-A- 5 643 738
- US-A- 5 677 195

## Beschreibung

### 1 Anwendungsgebiet der Erfindung

### 1.1 Hintergrund

Für die Grundlagenforschung in den Biowissenschaften und für die medizinische Diagnostik sowie einige andere Disziplinen ist die präzise Detektion biologisch relevanter Moleküle in definiertem Untersuchungsmaterial von herausragender Bedeutung. Dabei liegt die genetische Information in Form einer enormen Vielfalt von unterschiedlichen Nukleinsäuresequenzen vor, der DNA. Die Realisation dieser Information führt über die Herstellung von Abschriften der DNA in RNA meist zur Synthese von Proteinen, die ihrerseits häufig an biochemischen Reaktionen beteiligt sind.

Die Detektion von bestimmten Nukleinsäuren und die Bestimmung der Abfolge der vier Basen in der Kette der Nukleotide, die generell als Sequenzierung bezeichnet wird, liefert wertvolle Daten für Forschung und angewandte Medizin. In der Medizin konnte in stark zunehmendem Masse durch die in vitro-Diagnostik (IVD) ein Instrumentarium zur Bestimmung wichtiger Patientenparameter entwickelt und dem behandelnden Arzt zur Verfügung gestellt werden. Für viele Erkrankungen wäre eine Diagnose zu einem ausreichend frühen Zeitpunkt ohne dieses Instrumentarium nicht möglich. Hier hat sich die genetische Analyse als wichtiges neues Verfahren etabliert, z.B. für Infektionskrankheiten wie HIV und HBV, genetische Prädisposition für bestimmte Krebsarten oder andere Erkrankungen, Forensik und eine Vielzahl weiterer Anwendungsgebiete. In enger Verzahnung von Grundlagenforschung und klinischer Forschung konnten die molekularen Ursachen und (pathologischen) Zusammenhänge einiger Krankheitsbilder bis auf die Ebene der genetischen Information zurückverfolgt und aufgeklärt werden. Diese Entwicklung steht allerdings noch am Anfang, und gerade für die Umsetzung in Therapiestrategien bedarf es stark intensivierter Anstrengungen. Insgesamt haben die Genomwissenschaften und die damit verbundene Nukleinsäureanalytik sowohl zum Verständnis der molekularen Grundlagen des Lebens als auch zur Aufklärung sehr komplexer Krankheitsbilder und pathologischer Vorgänge enorme Beiträge geleistet.

Die weitere Entwicklung in der medizinischen Versorgung wird durch die Explosion der Kosten belastet, die mit entsprechend aufwendigen Verfahren verbunden sind. Hier muss nicht nur auf die Realisation der Möglichkeiten an diagnostischem und therapeutischem Nutzen gedrängt, sondern auch eine Integration in ein tragfähiges, finanzierbares Gesundheitssystem vorangetrieben werden.

Eine Anwendung entsprechender Technologien in der Forschung kann ebenfalls nur dann in breitem Umfang und auch im akademischen Bereich erfolgen, wenn die damit verbundenen Kosten reduziert werden.

### 1.2 Bedarf

Die Entwicklung der Genom- und Proteomwissenschaften und die Entschlüsselung des Erbgutes stehen noch am Anfang der Entwicklung, ebenso die Realisation des diagnostischen Potentials einer genetischen bzw. gentechnischen Analyse. Die bisher etablierten Verfahren sind meist arbeitsaufwendig und relativ ineffizient, was sich in Kosten und Kapazität z.B. an Informationsgewinn niederschlägt. Wichtigste Neuerung ist die Entwicklung von sog. Oligonukleotid-Arrays, bei denen eine sehr große Anzahl von relativ kurzen Oligonukleotiden definierter Sequenz auf einer festen Matrix (meist Silizium) angekoppelt sind und dadurch für eine parallele Hybridisierung komplementärer Sequenzen im Untersuchungsgut zur Verfügung stehen. Die aufwendige Herstellung und der hohe Preis lassen die Vermarktung als Massenprodukt zum gegenwärtigen Zeitpunkt aber nicht zu.

### 1.3 Anwendungsfelder

Für die in vitro Diagnostik und klinische Diagnostik soll durch deutlich preiswertere Systeme die Anwendung in der Routine möglich werden, z.B. für Infektionskrankheiten (HIV, HBV etc.) und deren Subtypen, für die Onkologie (Tumorfrüherkennung, Tumorklassifizierung, z.B. Typ und Status), und für die Bestimmung einer genetischen Prädisposition.

Für die biologische Grundlagenforschung, insbesondere die Genomik, ist die Erfassung von sehr vielen Messpunkten im untersuchten System, z.B. alle exprimierten Gene wünschenswert. Daraus ergibt sich ein enormer Erkenntnisgewinn in der biologischen Grundlagenforschung (Entwicklungsbiologie, Stammzellkultur, Tissue-engineering, Transplantationsmedizin, Regeneration) ableiten, der auch zu wichtigen Durchbrüchen in der Biomedizin und zu entsprechenden Anwendungen führen wird.

Wie für die Verwendung von DNA-Chips gezeigt wurde (Science 280:1077-1082), kann durch entsprechende biochemische Rahmenbedingungen in der Hybridisierung zwischen Punktmutationen in der Basenabfolge unterschieden werden. Mit dem hier beschriebenen System ist damit ein breit angelegtes Screening möglich, das für forensische Zwecke, z.B. zur Überführung von Straftätern oder zum Verwandtschaftsnachweis eingesetzt werden kann.

Durch diese Erfindung wird auch das rasche, kosteneffektive Analysieren von Lebensmitteln z.B. auf das Vorhandensein bestimmter Gene von pathogenen Keimen oder von genetisch veränderten Organismen hin möglich.

Ebenfalls von großer Bedeutung ist das Screening von medizinischen Produkten. Die Herstellung z.B. von Blutpräparaten ist immer noch mit einem hohen Aufwand- für die Sicherheitsvorkehrungen zur Reinheit verbunden. Ein zeit- und kosteneffektives Screening solcher Proben wird mit dieser Erfindung möglich werden, um z.B eine Kontamination mit infektiösem Material zu verhindern (HIV, HBV, HCV etc).

### 2. Stand der Technik

Bei BioChips handelt es sich um miniaturisierte hybride Funktionselemente mit biologischen und technischen Komponenten, z.B. an der Oberfläche eines Trägers immobilisierte Biomaterialien, die als spezifische Interaktionspartner dienen können (z.B. DNA-Oligonukleotide) und eine SiliziumMatrix. Meist sind diese Funktionselemente in Reihen und Spalten angeordnet, man spricht dann von BioChip-Arrays. Da Tausende von biochemischen Funkionselementen auf dem BioChip angeordnet sein können, müssen diese mit mikrotechnischen Methoden hergestellt werden.

Vor allem in den USA wird die Entwicklung von miniaturisierten BioChips mit enormen Mitteln vorangetrieben. Die wichtigsten in diesem Umfeld tätigen Firmen sind im folgenden aufgelistet:

Affymetrix, Beckman Instruments, Blue Chip Biosystems, Caliper Technologies, Cura-Gen, Genometrix, Gene Trace Systems, Hyseq, Incyte Pharmaceuticals, Molecular Tool, Nanogen, Pharmacia, Synteni, Third Wave Technologies, Vysis.

Bisher bekannte BioChips lassen sich nach folgenden Kriterien klassifizieren:
* Nachweisprinzip:
   - Chromatographische Verfahren
   - Interaktion von Analyten mit fester Phase, meist immobilisierter Interaktionspartner (z.B. Hybridisierung von Nukleinsäuren an DNA-Oligonukleotide).
* Detektionsverfahren (optisch, elektrisch).
* Markerbasierte Nachweisverfahren (z.B. Absorption, Fluoreszenz oder Lumineszenz) oder markerfreie Nachweisverfahren (Lichterzeugung zum Reaktionsnachweis).
* Zuordnung des Analyten zu seinem Träger [Festphase] (Array mit mehr als einem immobilisierten Interaktionspartner pro Träger oder Single mit nur einem immobilisierten Interaktionspartner pro Träger).
* Herstellungsverfahren (z.B. Oligonukleotide direkt auf dem BioChip lichtaktiviert synthetisieren, fertig synthetisierte Oligonukleotide spotten, Beads oder Tubes beschichten).
* Trägerarten (Glas-Chips, Kunststoff-Chips, Mikrotiterplatten, Tubes oder Beads).
* Präsentation zur Detektion (seriell, parallel).
* Optische Detektion (seriell im Scanner oder parrallel mit einer CCD-Kamera).

Unter den aufgeführten Firmen verwendet lediglich Affymetrix das Prinzip der Photolithographie für eine "high density" Erzeugung von DNA-Arrays auf einer planaren Oberfläche, wodurch sie die Parallelisierung der Detektion von Oligo-Sequenzen mit Abstand weitesten voran getrieben haben.

### GenChip von Affymetrix Inc., Santa Clara, Kalifornien:

Die Herstellung erfolgt durch in situ Synthese von DNA-Oligonukleotiden auf planaren Chips in hoher Dichte (Juli 98: bis 64.000 unterschiedliche Oligos auf 1 cm²). Das Herstellungsverfahren basiert auf der in der Halbleiter-Industrie verwendeten und optimierten Photolithographie, wobei eine lichtaktivierbare Bindung von Oligos an die Chipoberfläche, sowie an bereits vorhandene Oligos, verwendet wird. Die Herstellungsdauer beträgt aufgrund einer Vielzahl von Verfahrensschritten mehrere Stunden. Der Nachweis erfolgt durch serielle optische Detektion des planaren Chips in einem Fluoreszenzscanner. Die Hybridisierungsdauer der Probe auf einem Chip beträgt ca. 1.5 Stunden. Erste Produkte (Sequenzierchip für Tumormarker p53 Exons 2-11, Brustkrebsgen BRCA1 Exon 11, HIV GeneChip) sind bereits kommerziell erhältlich. Die Kosten liegen zur Zeit im Bereich mehrerer hundert Dollar für einen GenChip, zusätzlich wird noch eine Detektionseinheit benötigt.

US 5677195 (A) beschreibt ein Verfahren zur Synthese eines Polymerarrays auf einem Substrat. Dieses Array kann zum Screening verwendet werden, wobei zum Beispiel das Substrat einem markierten Rezeptor ausgesetzt wird, dessen Bindung an das Substrat lokalisiert werden kann.

US 5643738 (A) beschreibt ein Verfahren zur parallelen Synthese von Proteinen und Oligonukleotiden auf einem Substrat. Ein durch dieses Verfahren gebildetes Array kann zur klinischen Diagnose oder für Immunoassays verwendet werden.

WO 9813683 (A1) beschreibt eine Vorrichtung zur parallelen Detektion von Licht, welches von einem planaren Träger mit einer Vielzahl von Detektionsstellen emittiert wird oder diesen Träger durchstrahlt.

Weiterer relevanter Stand der Technik sind WO91/18276, EP-A-0 671 626 und EP-A-0 430 248.

### 3. Gegenstand der Erfindung und damit gelöste Aufgabe

Gegenstand der Erfindung ist ein Verfahren nach Punkt 14 zur integrierten Synthese und Analytbestimmung an einem Träger umfassend die Schritte
(a) Bereitstellen eines Trägerkörpers,
(b) Leiten einer Flüssigkeit mit darin enthaltenen Rezeptoren oder Bausteinen für die Synthese von polymeren Rezeptoren über den Träger,
(c) orts- oder/und zeitspezifisches Immobilisieren der Rezeptoren oder Rezeptorbausteine an jeweils vorbestimmten Positionen bzw. Bereichen auf dem Träger,
(d) gegebenenfalls Wiederholen der Schritte (b) und (c) bis die gewünschten Rezeptoren an den jeweils vorbestimmten Positionen bzw. Bereichen auf dem Träger synthetisiert worden sind,
(e) Inkontaktbringen des Trägers mit einer die zu bestimmenden Analyten enthaltenden Probe und
(f) Bestimmen der Analyten über deren Bindung an die auf dem-Träger immobilisierten Rezeptoren
dadurch gekennzeichnet, dass in einem Synthese/Analytenbestimmungszyklus mehrere Träger synthetisiert werden und eine direkte logische Verknüpfung zwischen den Ergebnissen der Analyse eines ersten Trägers und der Synthese des darauf folgend zu synthetisierenden Trägers erfolgt, wobei die Rezeptoren für einen nachfolgenden Träger auf Basis der Informationen aus dem vorhergehenden Träger synthetisiert werden.

Bevorzugte Ausgestaltungen dieses Verfahrens sind Gegenstand der Punkte 15 bis 25. In dieser Ausführungsform können auch planare Träger verwendet werden.

Gegenstand von Punkt 26 ist ein Träger für die Bestimmung von Analyten umfassend mindestens einen Kanal und vorzugsweise eine Vielzahl von Kanälen insbesondere Kapillarkanälen, wobei in den Kanälen eine Vielzahl von unterschiedlichen Rezeptoren immobilisiert ist. Vorzugsweise ist der Träger zumindest im Bereich der mit Rezeptoren zu bestückenden Bereiche optisch transparent.

Gegenstand der Erfindung ist weiterhin ein Reagenzienkit nach Punkt 28 umfassend einen Träger wie zuvor beschrieben und Bausteine für die Synthese von polymeren Rezeptoren auf dem Träger. Weiterhin kann der Reagenzienkit noch Reaktionsflüssigkeiten zur Synthese der Rezeptoren auf dem Träger enthalten.

Gegenstand der Erfindung ist auch eine Vorrichtung zur integrierten Synthese und Analytbestimmung an einem Träger nach Punkt 29 umfassend eine programmierbare Lichtquellenmatrix, eine Detektormatrix, einen zwischen Lichtquellen -und Detektormatrix angeordneten Träger sowie Mittel zur Zufuhr von Fluids in den Träger und zur Ableitung von Fluids aus dem Träger. Die programmierbare Lichtquellen- bzw. Belichtungsmatrix kann eine Reflexionsmatrix, eine Lichtventilmatrix, z.B. eine LCD-Matrix oder eine selbstemittierende Belichtungsmatrix sein. Bevorzugte Ausgestaltungen dieser Vorrichtungen ist Gegenstand der Punkte 30 und 31.

Schließlich noch ein Gegenstand der Erfindung ist die Verwendung des beanspruchten Verfahrens, Trägers, Reagenzienkits sowie der beanspruchten Vorrichtung zur Bestimmung eines Analyten in einer Probe. Bevorzugte Anwendungen sind Gegenstand der Punkte 33 bis 38.

Eine Ausführungsform der vorliegenden Erfindung stellt ein Verfahren und System zur zyklischen integrierten Synthese und Analyse dar, welches als ISA-System bezeichnet sein soll. Durch diese erfindungsgemäß bevorzugte unmittelbare Kopplung von Synthese und Analyse wird in einem zyklisch verlaufenden Verfahren eine gegenüber dem Stand der Technik deutlich verbesserte Hochdurchsatz-Bestimmung von Analyten ermöglicht. Dabei können die zu analysierenden Substanzen beispielsweise als Bruchstücke oder Fragmente einer größeren Molekülkette vorliegen.

Gemäß einer bevorzugten Ausführungsform der Erfindung wird eine direkte logische Verknüpfung zwischen den Ergebnissen der Analyse eines ersten Trägers und der Synthese des darauffolgend zu erstellenden Trägers bereitgestellt, wobei sich der Informationsgewinn aus einem vorhergehenden Zyklus in einen nachfolgenden Zyklus übertragen lässt. Auf diese Weise wird ein Lernen des Analysesystems schrittweise entwickelt.

Die besagte zyklisch verlaufende Abfolge von Synthese, Sequenzvergleich, Analyse der Vergleichsergebnisse und wiederum erfolgender Synthese von Rezeptoren am Träger kann beliebig oft - bis zum Erreichen eines gewünschten, beliebig zu wählenden Abbruchkriteriums - wiederholt werden.

Durch die Rückkopplung und den damit verbundenen Lernprozess von vorhergehendem Zyklus sind das erfindungsgemäße Verfahren und die Vorrichtung auch für die Erforschung sehr großer und komplexer Analyt-Molekülketten, z.B. zum Sequenzieren individueller Genome, wie dem menschlichen Genom, geeignet. Der Zeitaufwand verbessert sich dabei gegenüber dem Stand der Technik mindestens um das hundertfache, eher um das tausendfache und -potentiell um das 10.000-fache.

Das- Verfahren kann zur "Neusequenzierung" nicht bekannter Nukleinsäuresequenzen (DNA, cDNA, RNA) einschließlich deren räumlichen Zuordnung, respektive Kartierung eingesetzt werden. Mit diesem Vorgehen ist es möglich, ein individuelles Genprofil jedes Individuums und jeder Spezies zu erstellen, sei es durch Sequenzierung von Teilen des Genoms oder des ganzen Genoms.

Weiterhin kann das Verfahren zur "Resequenzierung" von Nukleinsäuresequenzen eingesetzt werden, d.h. zum Vergleich von bereits bekannten Sequenzen (repräsentiert in Form der Rezeptorsonden) mit unbekannten Sequenzen in der zu untersuchenden Probe. Die bekannten Sequenzen werden dazu gezielt und der Fragestellung entsprechend ausgewählt.

Die beschriebene Resequenzierung erlaubt es dem Anwender, individuelle polymere Rezeptoren vor Ort auf dem erfindungsgemäßen Träger ausgehend von einem neutralen Träger zu erzeugen und anschließend sofort eine Analyse der zu untersuchenden Probe durchzuführen. Durch diese Möglichkeit entsteht eine maximale Variantenvielfalt der Rezeptoren bei minimalem Platzbedarf.

Durch Kombination von Neu- und Resequenzierung, können diagnostische Tests oder Medikamente kurzfristig an die Bedürfnisse eines Individuums angepasst werden.

Als weiteres wichtiges Anwendungsgebiet können außerordentlich flexibel Expressionsmuster analysiert werden. Die entsprechenden Rezeptoren bzw. Polymersonden werden dazu in aller Regel anhand bekannter Sequenzen ausgewählt. Der Einsatz des Verfahrens zur Bestimmung der Genexpression kann auch im Kontext von Hochdurchsatz-Screening erfolgen.

Darüber hinaus sind mit unterschiedlichen -natürlich vorkommenden und künstlichen Rezeptorsonden unterschiedliche Ansätze für Screening-Verfahren und den Aufbau und die Analyse von Substanzbibliotheken denkbar. Dies kann z.B. im Zusammenhang mit der Suche nach und der Charakterisierung von pharmakologisch aktiven Substanzen erfolgen.

Die Anwendungsfelder für das erfindungsgemäße Verfahren und die erfindungsgemäße Vorrichtung zur zyklisch integrierten Synthese und Bestimmung von Analyten sind breitgefächert und erstrecken sich prinzipiell auf alle Anwendungen der Analyse wie Gas-Chromatographie, Dünnschicht-Chromatographie,Gel-Elektrophorese,Kapillar-Elektrophorese, Massenspektrometrie etc. Gleiches gilt prinzipiell für alle Anwendungen hochparalleler Festphasen-Analyse.

Die Notwendigkeit, fertige und komplexe polymere Rezeptoren zu lagern, entfällt vollständig. Darüber hinaus gibt es keine physikalische Beschränkung bezüglich der Anzahl und Auswahl der Rezeptoren. Die benötigte Anzahl der Rezeptoren kann über mehrere Reaktionsträger bzw. mehrere Zyklen in einem Reaktionsträger verteilt werden, da die einzelnen Rezeptoren für die logische Auswertung der Vergleichsergebnisse keinen Ortsvorgaben unterliegen.

Ein neuer "Träger" als Basis für die Verwendung einer vorzugsweise lichtgesteuerten Synthese einzelner Basen (G, A, C und T) oder ganzer Oligonukleotide (Basen- Sequenzen) zur Bildung einer hochparallelen, -planaren und -dichten Anordnung (Array) dieser Oligonukleotide in einer festen Trägermatrix (Chip) wird beschrieben.

Der neue BioChip, der "opto-fluidische Mikroprozessor", enthält eine Struktur aus Mikrokanälen, vorzugsweise Kapillaren in einem zumindest teilweise durchsichtigen und vorzugsweise flachen Körper. Die flüssigen Einsatzstoffe werden bei der Rezeptorsynthese oder -immobilisierung durch die Kanäle im Träger geführt und binden, lokal aktiviert, an den Kanalwänden. Damit werden die technischen Vörraussetzungen für eine schnelle, effiziente und damit kostengünstige Herstellung geschaffen, was den breiten Einsatz dieser Träger ermöglichen wird. Dichte und Parallelität liegen in der gleichen Größenordnung wie bei Konkurrenztechniken, mit mehreren hunderttausend definierten Oligonukleotiden auf einem Träger. Der Vorteil der neuen Technik liegt in den günstigeren physiko-chemischen Eigenschaften der Strömungs- und Benetzungsvorgänge in den Kanälen im Vergleich mit einer einheitlichen Oberfläche.

Die Chip-Produktion besteht aus der Erzeugung eines vorzugsweise mit Mikrokanälen versehenen Trägerkörpers aus einem geeigneten, lichtdurchlässigen Material sowie dem biochemischen Beschichtungsvorgang vorzugsweise an den Wänden der einzelnen Mikrokanäle, so dass anschließend die polymeren Rezeptoren, z.B. Oligonukleotide, in den Kanälen synthetisiert werden können. Hierbei werden in den einzelnen Kanälen im Träger, mittels Photoaktivierung durch eine geeignete Lichtquelle einzelne Rezeptorbausteine, oligomere Synthone (z.B. Di-, Tri-, Tetra- oder Pentanukleotide) oder ganze Basen-Sequenzen (Oligos) ortsspezifisch angelagert. Dadurch entstehen in jedem Kanal eine Vielzahl an Rezeptor-bestückten Bereichen (spezifische Bindungs- bzw. Hybridisierungsstellen), wobei jeder Bereich aufgrund seiner individuellen Rezeptor-Sequenz-Kombination für die Bindung und anschließende Detektion eines spezifischen Analyten, z.B. eines DNA-Fragments dient. Die Bereiche sind in einer Dimension des planaren Trägers durch die Wände der Kanäle voneinander getrennt und entlang der einzelnen Kanäle wird zwischen zwei benachbarten Bereichen bei der photoaktivierten Bindung ein entsprechender Freiraum gelassen. Es entsteht ein hoch paralleler, hoch integrierter Array von spezifischen Rezeptoren. Aufgrund der Möglichkeit des Multiplexens von-Oligo-Sequenzen und parallelen Kanälen (Details siehe Kapitel 5) ist eine Reduktion der Herstellzeiten auf 1/4 bei der Verwendung einfacher Basen, 1/8 bei Dinukleotiden und auf 1/16 bei Trinukleotiden durch entsprechendes Multiplexing der Oligos (Einsatzstoffe) und der zu benetzenden Kanälen möglich. Damit wird auch eine flexible Anpassung an Kundenwünsche, der "massgeschneiderte" BioChip, möglich. Diese systematische Beschleunigung ist in planaren Systemen (planaren Chips) nicht möglich.

Für die Analyse wird das Untersuchungsmaterial (z.B. DNA, RNA in Lösung) durch die Kanäle geführt und erhält Gelegenheit zur Bindung an die Rezeptoren, z.B. durch Hybridisierung an komplementäre Stränge, sofern diese vorhanden sind. Zur Detektion und Auswertung der jeweiligen Analytbindung, z.B. einer DNA-Hybridisierung, werden vorzugsweise hochauflösende, parallele CCD- Chips verwendet. Die Bindung des Analyten an den immobilisierten Rezeptor wird durch geeignete aus dem Stand der Technik bekannte signalgebende Gruppen, z.B. Lichtemittierende Gruppen. Es sind aber auch neue Detektionsverfahren anwendbar. Bei der Detektion kann auf optisch abbildende Linsensysteme verzichtet werden, wenn die Größe der Kanäle so gewählt wird, dass jeder Messpunkt eine ausreichende Anzahl Pixelelemente des Detektors, z.B. eines CCD-Chips überdeckt. Durch diese direkte Nutzung (keine Optik) hoch paralleler CCD-Matrix Chips mit einer großen Anzahl (derzeit 16 Mio. Pixel pro 1 cm²; Stand der Forschung: 80 Mio. Pixel pro 1 cm²) an Pixeln (optischen Sensoren) ist es möglich eine Vielzahl von Lichtsignalen parallel zu detektieren (siehe BioScanner der Firma Genometrix). Damit wird versucht, auch bei der Detektionseinheit statt teurer optischer Anordnungen auf ein in großer Stückzahl und zu niedrigen Preis gefertigtes High-Tech Produkt zurückzugreifen.

Durch die Erfindung werden somit wesentliche Anforderungen in der DNA-Analytik abgedeckt, nämlich simultane-Bestimmung einer Vielzahl an DNA-Sequenzen (erreicht durch hoch integrierte, miniaturisierte-Träger und eine hochauflösende optische Detektion), Bereitstellung kostengünstiger Tests (Multiplexing in der Produktion, billige "Disposable"- Träger zum Beispiel aus Spritzguss, schnelle Synthese bei der Produktion), eine schnelle Durchführung bei der Analyse aufgrund kleinerer Volumina und günstiger Benetzungsvorgänge, Reduktion der Einsatzstoffe durch die Strömungsgeometrie des Träger etc.), schnelle Auswertung (erreicht durch die parallele optische Auswertung in planarer Anordnung [DNA-Chip Array]), ein kostengünstiges Analysesystem (erreicht durch den Verzicht auf teure, mikrosystemtechnische und optische Komponenten) und die Sicherstellung der Qualität sowohl bei der Produktion, als auch bei der Analyse (erreicht durch definierte Strömungsvorgänge im Träger).

Die Verwendung der Photoaktivierung von chemischen Reaktionen im Bereich der Träger-Synthese kommt insbesondere in Kombination mit der Technologieplattform des opto-fluidischen Mikroprozessors zusammen mit einer programmierbaren Lichtquellenmatrix zum Durchbruch, da hierdurch die Produktionskosten für einen einzelnen Träger, bei gleichzeitiger Qualitätsverbesserung, um den Faktor 10-100 reduziert werden können. Dadurch wird zum ersten mal eine kostengünstige, massiv parallele, hoch integrierte und gleichzeitig einfach miniaturisier- und automatisierbare DNA-Chip-Technologie zur Verfügung gestellt.

Trotz der komplexen Datenauswertung bedarf es nur eines Minimums an unterschiedlichen Hardware-Komponenten, da die Trägerkörper, die entweder pro Zyklus oder auch nur bei Verschleiß gewechselt werden müssen, zunächst - vor Beginn der Rezeptorsynthese - alle identisch sind. Alle Individualität ergibt sich erst aus der spezifischen Rezeptorsynthese und aus der schrittweise durch die Analyse gewonnene Information, die nach dem Synthese/Analysezyklus wieder in Information überführt wird, so dass die Individualität, d.h. die kennzeichnenden Merkmale der biologischen/chemischen Materie wiederum nur in Form elektronischer Daten vorliegen.

### 4. Grundzüge des Lösungsweges

Der prinzipielle Lösungsweg in diesem System basiert auf der schrittweisen biochemischen Synthese von Rezeptoren an die Oberflächen einer Vielzahl von Kanalwänden auf einen Träger. Diese Kanäle sind auf dem Träger, z.B. einem kleinen planaren Chip, angeordnet. Die Synthese erfolgt mit den entsprechenden Basen oder mehrbasigen Oligonukleotiden (Basen-Sequenzen) über eine lichtaktivierte ortsspezifische Bindung. Die Benetzung dieser spezifisch "gelabelten" Kanäle mit den zu untersuchenden DNA-Analyten und der anschließenden Detektion der Bindungsreaktion über geeignete signalgebende Gruppen schließt einen Verfahrenszyklus ab.

### 4.1 Mikrostruktur als Trägermatrix

Die Träger-Synthese umfasst die Bereitstellung des Trägerkörpers, der vorzugsweise aus einem geeigneten, lichtdurchlässigen Material besteht, sowie dem biochemischen Erzeugen von Rezeptoren an den Wänden der einzelnen Kanäle. Die spezifische Synthese der Rezeptoren kann entweder direkt bei der Herstellung des Trägerkörpers oder erst beim Anwender erfolgen.

Für die Trägerkörper können unterschiedliche Materialien (z.B. Glas, Silizium, Keramik, Metall oder Kunststoff) verwendet werden. Wichtig ist, dass die Wände der Kanäle sowohl für die Anregungswellen bei der lichtaktivierten Synthese sowie die Lichtwellen (ggf. Anregung und Reaktionssignal) bei der anschließenden Detektion (Analyse) gut durchlässig sind. Je nachdem welches Material eingesetzt wird, müssen die Wände der Kanäle mit einem reaktionsfähigen Material beschichtet werden, damit die Rezeptoren oder Rezeptorbausteine an der Oberfläche binden können.

Die Geometrie der Träger entspricht beispielsweise einer "Scheckkarte", wobei die Größe der von den Kanäle bedeckten Fläche von dem zur Detektion verwendeten CCD-Chip bestimmt wird. Für die Kanäle im Träger sind unterschiedliche Herstellverfahren einsetzbar. Hierbei ist der Einfluss der Querschnittsgeometrie der Kanäle zu berücksichtigen, welcher großen Einfluss auf die entstehenden strömungstechnischen Kräfte und die Möglichkeit der Reinigung der Kanäle hat. Als Herstellverfahren kann man zum Beispiel Laser, Fräsen, Ätztechniken oder Spritzguss verwenden.

Bei der Anordnung der Kanäle in der Ebene sind die folgenden Aspekte zu berücksichtigen: Verwendet man eine Vielzahl an parallelen Kanälen, so kann man die Zeiten bei der Synthese minimieren, allerdings ist die Benetzung bzw. Befüllung des einzelnen Kanals entsprechend komplex. Hat man im anderen Extrem nur einen einzigen langen Kanal, so ist die Synthese entsprechend langsam, da das Multiplexing von Kanälen zu Basen oder ganzen Oligos nicht verwendet werden kann und alle Vorgänge nur seriell nacheinander ablaufen können. Der Vorteil nur eines Kanals liegt in der Analyse, wo die Probe an jedem Messpunkt in allen Kanälen vorbeiströmt.

### 4.2 Synthesezyklus im Träger

In einem Trägerkörper werden die zur Beschichtung mit Rezeptoren vorgesehenen Positionen (Reaktionsbereiche) durch Kanäle aus Behältern über Zuleitungen, Ventile und Fittings mit einem oder mehreren Fluiden befüllt. Mit Hilfe einer aus der deutschen Patentanmeldung 198 39 254.0 bekannten Lichtemissions/Detektionseinrichtung, die vorzugweise eine programmierbare Lichtquellen- bzw. Belichtungsmatrix darstellt, wie sie in der deutschen Patentanmeldung 199 07 080.6 beschrieben ist, können ausgewählte Positionen bzw. Bereiche des Trägers belichtet und auf diese Weise die individuelle Synthese von Rezeptoren gesteuert werden, wobei der Träger in diesem Zusammenhang einen opto-fluidischen Mikroprozessor darstellt. Anstelle einer Belichtung ist auch eine individuelle fluidische Aktivierung der ausgewählten Reaktionsbereiche möglich. Nach Abschluss der Reaktion werden die Reaktionsbereiche gespült und neu gefüllt, wonach sich wiederum ein Aktivierungszyklus anschließt. Der Verlauf der Rezeptorsynthese kann mittels geeigneter Detektionseinrichtungen verfolgt und gesteuert werden.

Sobald die Synthese der Rezeptoren abgeschlossen ist, werden die Reaktionsbereiche gereinigt und stehen dann für ein Analytbestimmungsverfahren zur Verfügung.

### 4.3 Nukleinsäureanalytik mittels Oligo-chips - Grundprinzip

Wie bereits für mehrere Anordnungen gezeigt (z.B. Molekular Medicin Today, 9/97, S. 384-389; Trends in Biotechnology, 11/97, S. 465-468) kann die Hybridisierung von Nukleinsäuresträngen an eine meist kurze komplementäre Sequenz, ein sog. Oligonukleotid oder Oligo, für die Sequenz-Analyse verwendet werden. Dazu werden hochdichte Anordnungen synthetischer Oligonukleotide auf einer festen Matrix erzeugt und erlauben multiple parallele Hybridisierungsexperimente. Führendes Verfahren (August 98) ist eine photolithographische und damit lokale Aktivierung von Synthese-Vorstufen. In Anlehnung an die aus der Mikroelektronikherstellung entlehnte Technik werden die parallelen Anordnungen als Chips bezeichnet.

Durch eine massive Erhöhung der Zahl an Reaktionsbereichen ('Messpunkten'), d.h. definierten Oligos an definiertem Ort, wird eine enorme analytische Kapazität geschaffen.

Die zu untersuchende Probe enthält normalerweise DNA oder RNA. Diese muss eventuell isoliert und in einem Amplifizierungssohritt (z.B. PCR) vermehrt werden und erhält dabei eine Markierung, z.B. ein Farbstoff-, Fluoreszenz- oder Lumineszenzlabel.

Durch ausreichend viele Rezeptor-bestückte Bereiche (Reaktionsbereiche) ist auch eine Sequenzierung eines DNA Moleküls- möglich (Sequencing-by-Hybridization SBH, siehe BioTec 3/98, S. 52-58), andere Anwendungen zeigen die Bestimmung von Punkmutations-Polymorphismen (d.h. Unterschiede zwischen Individuen in einzelnen Basen in einem definierten DNA Abschnitt) und erlauben u.a. eine Identifizierung von solchen Polymorphismen bei hunderten von Probanden parallel (Science 280, 5/98, S. 1077-1082).

Erstmals wird auch die Untersuchung von ganzen Genomen und des Gen-Expressions- Status ganzer Zellen möglich (z.B. Proc.Nat.Acad.Sci.USA 95, 3/98, S. 3752-3757).

Die hier beschriebene Erfindung lässt demnach die Anwendung einer Vielzahl von etablierten Verfahren zur Untersuchung von Nukleinsäuren und genetischem Material zu. Damit ist gleichzeitig ein starker Anstieg solcher Anwendungen und damit ein enormer wissenschaftlicher Fortschritt verbunden, da erwartet wird, dass der opto-fluidische Mikroprozessor eine solche Technologie flexibler als die vorhandenen Verfahren und zu deutlich niedrigeren Kosten bereitstellt.

### 4.4 Lichtaktivierte Synthese von Oligonukleotiden und Peptiden auf dem Träger

Beim Aufbau von Rezeptoren auf dem Träger werden in den einzelnen Bereichen mittels Photoaktivierung durch eine geeignete Lichtquelle Rezeptorbausteine, z.B. einzelne Basen (G, A, C, T) oder Oligonukleotid-Sequenzen (vorzugsweise etwa 2 bis 4 Basen lang) ortsspezifisch angelagert. Die Kanäle werden sequentiell mit den Synthesebausteinen, z.B. G, A, C und T, gefüllt und entlang der Kanäle ortsspezifisch mit hochauflösendem Licht bestimmter Wellenlänge und Intensität bestrahlt. Zwischen den Beschichtungszyklen werden die Kanäle entsprechend gespült, um nicht gebundene Rezeptorbausteine zu beseitigen.

Hierdurch entstehen in jedem Kanal eine Vielzahl an Reaktionsbereichen (spezifische Bindungs- bzw. Hybridisierungsstellen), wobei jeder Reaktionsbereich aufgrund seiner individuellen Rezeptor-Sequenz für die Bindung und anschließende Detektion eines spezifischen Analyten, z.B. eines DNA-Fragments dient. Die Reaktionsbereiche sind in der einen Dimension des planaren Trägers durch die Wände der Kanäle voneinander getrennt und in der zweiten Dimension, entlang der einzelnen Kanäle, wird zwischen zwei benachbarten Reaktionsbereichen bei der Photoaktivierung ein entsprechender Freiraum gelassen.

Die Photolithographie kann auch weiterhin für die lichtaktivierte Bindung der Rezeptorbausteine verwendet werden. Es können aber auch andere Verfahren eingesetzt werden.

Besonders bevorzugt wird ein Belichtungsverfahren unter Verwendung einer programmierbaren Lichtquellenmatrix, z.B. einer selbstleuchtenden Lichtquellenmatrix, einer Lichtventilmatrix oder einer Reflexionsmatrix durchgeführt, deren Matrixpunkte bzw. Lichtquellenelemente gezielt steuerbar sind, insbesondere hinsichtlich der Intensität und gegebenenfalls Farbe des Lichtes. Mit einer solchen Matrix können also jeweils benötigte zweidimensionale Belichtungsmuster auf einfache Weise, insbesondere rechnergestützt erzeugt werden. Die bevorzugte Photoaktivierung der Oligos bei der Herstellung des Trägers erfolgt direkt durch die Belichtungsmatrix. Die hierfür benötigte Wellenlänge von beispielsweise 365 nm (oberer UV-Bereich nahe dem-sichtbaren Licht) lässt sich mit allen Varianten der programmierbaren Lichtquellenmatrix steuern.

Auf entsprechende Weise können auch Rezeptoren aus Aminosäureoder/und Peptidbausteinen aufgebaut werden.

### 4.5 CCD-Chip-Detektion der spezifischen Nachweisreaktion

Wie beschrieben soll die Bindung eines DNA-Analyten direkt oder indirekt zu einem nachweisbaren Signal, z.B. zu einem Lichtsignal führen. Dies kann beispielsweise durch Absorbtion, ein Anregungslicht (Fluoreszenz) oder durch Photonenemission (Lumineszenz) erfolgen. Zur Signaldetektion wird vorzugsweise ein CCD-Chip verwendet, der vorzugsweise direkt unter dem Träger plaziert wird. Die Anregungslichtquelle wird vorzugsweise über dem Träger plaziert und es wird entsprechend im Durchlichtverfahren gemessen.

Jedes Lichtsignal kann auf dem CCD-Chip erfasst werden, und zwar nach Intensität und bei Bedarf auch nach Wellenlänge (Farbe) differenziert. Das aufgenommene Spektrum kann qualitativ oder quantitativ ausgewertet werden. Zudem lässt die Unterscheidung von Wellenlängen und Intensitäten auch eine Unterscheidung von Signalquellen zu.

Die Anregungslichtarten für das Nachweisverfahren müssen je nach Anforderungen monochromatisch (z.B. Laserlicht für Fluoreszenzanregung) oder heterogen (z.B. Weißlicht für Absorptionsmessung) gewählt werden.

### 5. Verbesserungen und Vorteile gegenüber vorhandenen Systemen

Durch die neuen Träger werden die nachfolgend aufgeführten Nachteile von maskenbasierten Photolithographieverfahren oder dem in situ-Spotten überwunden.
* Das Prinzip der flächigen Benetzung der gesamten Chipoberfläche mit Fluid erlaubt keinerlei Multiplexing in der Produktion. So erhöht sich die Zahl der Hesrstellungszyklen für 20 Basen lange Oligos bei einer Verwendung von Dinukleotiden (4² = 16 Möglichkeiten) von 4 x 20 = 80 Hybridisierungsschritten auf 16 x 10 = 160, was eine Verdoppelung bedeutet. Das Gleiche gilt natürlich auch für die zwischengelagerten Waschzyklen.
* Die Synthese der photoaktivierbaren Basen an die planare Chipoberfläche, ebenso wie die benötigten Waschschritte bei der Chipherstellung sind, außer durch platz- und handhabungsintensive Tauchvorgänge (Chip wird in Flüssigkeit getaucht) oder flüssigkeitsintensive Spülvorgänge entlang der Oberfläche (z.B. Zentrifugationsprinzip aus der Halbleitertechni), nicht realisierbar, was von der Geräteentwicklung her gesehen ein sehr großes Miniaturisierungs- und Automatisierungshemmnis darstellt.
* Bei der anschließenden DNA-Sequenz-Detektion ist eine gleichmäßige Verteilung der Probe auf der Chipoberfläche aufwendig (keine einfachen und damit zuverlässigen Mischverfahren möglich) und es Bedarf einer entsprechend großen Menge an Proben-Fluid. Die Suche nach einem seltenen Ereignis in der Probe ist nicht möglich, da ein ausreichender Kontakt aller Probenbestandteile mit allen spezifischen Messpunkten nicht gewährleistet werden kann.

### 5.1 Reduktion der Produktionszeiten durch Multiplexing beim Synthetisieren

Der wesentliche Fortschritt der neuen Träger liegt in der Möglichkeit der drastischen Reduktion der Herstellzeiten bei der individuellen Synthese der Rezeptor-bestückten Träger durch ein entsprechendes Multiplexen zwischen Rezeptorbausteinen als Einsatzstoffen und den Kanälen.

Für die ortsspezifische Erzeugung einer Vielzahl an unterschiedlichen Rezeptorsequenzen, z.B. Basen-Sequenzen einer bestimmten Länge (z.B. 20 Basen) auf einer planaren Oberfläche mittels örtlich hochauflösender Photoaktivierung benötigt man in jeder Ebene (Rechenbeispiel: 20 Basen in jeder Basen- Sequenz) des DNA-Chip-Arrays 4 (bedingt durch die vier verschiedenen Basen) Synthesezyklen. Für 20 Basen- Ebenen sind es folglich 4 x 20 = 80 Zyklen. Verwendet man auf der gleichen Oberfläche Dinukleotide (2 Basen) so entstehen 2 Ebenen auf einmal, allerdings sind für diese zwei Ebenen 4² = 16 Synthesezyklen notwendig. Für 20 Ebenen werden folglich 10 x 16 = 160 Synthesezyklen anstelle von 80 Zyklen benötigt, was eine Verdoppelung der Produktionszeiten bedeutet. Bei der Verwendung von Trinukleotiden (3 Basen) verstärkt sich dieser Effekte auf mehr als die fünffache Anzahl an Zyklen. Somit ist bei einer einfachen planaren Oberfläche die Verwendung von einzelnen Basen als die schnellste Möglichkeit zur photoaktivierten DNA-Chip Erzeugung gegeben. Es besteht keine Möglichkeit die Anzahl an Synthesezyklen zu reduzieren.

Bei der Synthese des opto-fluidischen Trägers besteht im Unterschied hierzu die Möglichkeit, die Einsatzstoffe, sprich die Basen oder die unterschiedlichen Varianten der Di- (4² = 16 Kombinationen) oder Trinukleotide (43 = 64 Kombinationen) auf verschiedene Kanäle zu verteilen. D.h., zumindest in den unteren - "trägernahen" - Ebenen wird je Kanal nur immer eine Base bzw. eine der möglichen Basen-Sequenzen eingebracht. Je nach Festlegung der insgesamt zu erzeugenden Basen-Sequenzen in den Kanälen des Trägers kann es sein, dass in den oberen Ebenen dieses Prinzip teilweise aufgehoben werden muss, sprich für eine Basen-, Di- oder Trinukleotid- Ebene muss mehr als eine Base oder Oligo durch einen der Kanäle fließen. Dadurch erhöht sich auch hier die Anzahl der Synthesezyklen ggf. wieder etwas. Insgesamt bleibt jedoch eine sehr große Reduktion der Herstellzeiten auf theoretisch 1/4 der Zyklen bei einfachen Basen, 1 /8 der Zyklen bei Dinukleotiden und auf 1 /16 der Zyklen bei Verwendung von Trinukleotiden als Einsatzstoffe bei der RezeptorSynthese (und so weiter bei längeren Oligos). Die Anzahl der für einen spezifischen Träger benötigten Zyklen ist für jeden Träger individuell und kann nur als statistischer Mittelwert angegeben werden, wenn die Anzahl an Reaktionsbereichen auf bzw. in dem Träger, die Anzahl an parallelen Kanälen und die Länge der auf dem Träger zu synthetisierenden Oligos vorgegeben ist. Die Optimierung der Synthesezeiten eines Trägers soll mittels eines zu entwickelnden Softwaretools (z.B. CAMS Computer Aided Multiplexing Synthesis) erfolgen, welches in die Steuerung des zu entwickelnden Analysesystems bzw. in den angekoppelten Rechner integriert wird.

### 5.2 Reduktion der Einsatzstoffe und Qualitätssicherung

Die Verwendung von Kanälen reduziert die benötigte Fluidmenge und erhöht gleichzeitig die Qualität sowohl bei der Träger-Synthese, als auch bei der anschließenden Detektion einer Probe, im Vergleich zur Verwendung einer einfachen Fläche sehr stark. So ist das gleichmäßige Benetzen von Kanälen strömungstechnisch sehr einfach, verbraucht wenig Fluid und ist daher sehr leicht miniaturisier- und automatisierbar. Dies gilt insbesondere auch für die benötigten Waschvorgänge der Kanäle mit einer ausreichenden Qualität.

Durch die Wände der Kanäle, welche im Prinzip den Zwischenraum zwischen zwei Reaktionsbereichen im Träger-Array bedecken, wird das benötigte Fluid bereits um 50% reduziert. Dies gilt sowohl für das Beschichten des Trägers in der Produktion, das Synthetisieren der Rezeptoren als auch für den "Probenauftrag" für die Analyse. Eine weitere Reduktion der Fluidmengen erfolgt durch die gute Benetzung der Kanalwände durch ein durchströmendes Fluid und vor allem durch die effektiven Waschvorgänge, welche zum Beispiel durch "reinigende" Gasblasen in den Kanälen stark verbessert werden können. Eine gute, statistisch ausreichende Verteilung der Probe auf einer Fläche ist dagegen nur mit einer sehr großen Probenmenge realisierbar.

Ein weiterer Vorteil der Kanäle liegt in den kürzeren Zykluszeiten, welche durch die kleineren Fluidvolumina und die damit verbundenen schnelleren chemischen Reaktionen und Abläufen entstehen. Dies hat sowohl kürzere Synthese- als auch Hybridisierungszeiten zur Folge.

Zusätzlich wird hierdurch eine deutliche Fehlerreduktion sowohl bei der Produktion wie bei der Detektion erzielt, was die Zahl der auswertbaren Messungen pro Material- und Zeiteinsatz weiter erhöht und die Grundlage für eine Qualitätssicherung bildet, welche auf genau definierbare und reproduzierbare Strömungsvorgänge aufbaut.

Die einfache Miniaturisierung und Automatisierung der Abläufe in den neuen Trägern bilden die Basis für eine einfache Miniaturisierung und Automatisierung des gesamten neuen Analysesystems, welches auf den Trägern aufbaut.

### 5.3 Dreidimensionale Reaktionsoberflächen

Durch eine geeignete Auslegung der Querschnittsgeometrie der einzelnen Kanäle lässt sich die nutzbare Reaktionsoberfläche vergrößern. Die Größe dieser Fläche ist für die Anlagerung der Oligos bei der Produktion ebenso von Bedeutung wie für die Anlagerung der vorbeiströmenden DNA-Fragmente aus der Probe und der daraus resultierenden Intensität der Lichtsignale bei erfolgter Hybridisierung.

So hat ein rechteckiger Kanal, gleiche Höhe wie Breite vorausgesetzt, bei einer Nutzung der Wände und der Decke die vierfache Reaktionsoberfläche bei einer identischen Grundfläche, sprich dem gleichen Platzbedarf in den zwei Dimensionen eines planaren Trägers. Selbst wenn man die Kanäle, aus strömungstechnischen Anforderungen heraus, innen rund auslegt (zum Beispiel bessere Reinigungsmöglichkeiten durch Gasblasen im Kanal), bleibt noch eine etwa dreifache Reaktionsoberfläche im Vergleich mit einer planaren Oberfläche. Durch die Nutzung dieser dreidimensionalen Strömungsgeometrie kann der Einsatzstoffbedarf (Produktion und Analyse) weiter reduziert werden.

Ein anderer Effekt lässt sich ebenfalls durch die Querschnittsgeometrie der Kanäle beeinflussen: Die Lichtbrechung am Übergang vom Innenraum der Kanäle in das umgebende Material des Trägers. So hat jede Krümmung entweder einen fokussierenden oder streuenden Einfluss auf die Ausbreitungsrichtung des Lichtes. So kann man beim Träger durch eine entsprechende Wahl 1 der Ober- und Unterseite der Strömungskanalgeometrie die Lichtwege optimieren.

### 5.4 Parallele CCD-Chip Detektion

Das Messen der Lichtsignale aller Reaktionsbereich des Trägers "auf einmal" nutzt das ständig wachsende Potential der hochauflösenden CCD-Kamera Chips. Diese erlauben die Detektion aller Lichtsignale zum Reaktions- bzw. Hybridisierungsnachweis in einem einzigen Messvorgang. Hierfür stellen aktuelle Farb-CCD-Chips auf einer Fläche von 40 x 40 mm etwa 3000 x 3000 Pixel mit einer Pixelgröße von etwa 10 × 10 µm zur Verfügung. Der Stand der Forschung ist bereits bei entsprechenden CCD-Chips mit ca. 4000 x 6000 Pixeln. Die Signaldetektion erfolgt in Bruchteilen einer Sekunde für alle Pixel synchron. Damit ergibt sich auch für die beschriebene Applikation der CCD-Chip Technologie ein großes Wachstumspotential und die parallele Detektion von 10⁶ individuellen Reaktionsbereiche im Träger ist technisch machbar. Dadurch werden die zeitaufwendigen Scann-Vorgänge herkömmlicher Systeme vermieden und die reine Messzeit reduziert sich auf ein Minimum, welche im Verhältnis zu anderen Verfahrensschritten völlig bedeutungslos wird.

Das Bearbeiten der anfallenden Datenmengen ist, durch die Entwicklung der Leistungsfähigkeit bei gleichzeitigem Preisverfall von modernen Rechnersystemen, problemlos möglich.

### 5.5 Direktdetektion ohne Optik

Die direkte Detektion der -Lichtsignale, ohne Optik, durch einen CCD-Chip hat den Vorteil einer wesentlich niedrigeren Energiemenge, welche das Licht für eine fehlerfreie Detektion benötigt. Eine solche Anordnung soll - in einem anderen Zusammenhang untersucht - lediglich 10% der Anregungslichtmenge einer vergleichbaren Anordnung mit einer Optik verbrauchen. Anders ausgedrückt, die Optik schluckt 90% der Lichtenergie. Durch die geringere Lichtintensität werden unerwünschte Streulichteffekte im - die Kanäle umgebenden - Träger, ebenso wie eine eventuelle notwendige Kühlung der verwendeten Lichtquelle, stark reduziert. Außerdem bedeutet der Wegfall einer Optik eine große Platzersparnis sowie eine Verringerung der Herstellkosten für die Detektionseinheit.

Aufwendige Bewegungseinrichtungen für den Träger oder die Detektionseinheit, wie sie in Scannern notwendig sind, entfallen ebenfalls völlig. Die vorgegebenen Abmessungen der CCD-Chips (mehrere cm²) ermöglichen die Verwendung einer sehr großen Zahl an parallelen Kanälen (mehrere 100) mit einer moderaten Kanalgröße (im 10-100 µm Bereich).

### 5.6 Disposable Träger

Die Träger können als einfache Disposables (Einweg-Chips) ausgeführt werden. Prinzipiell sind sowohl Glas-, Silizium-, Metall-, Keramik- oder Kunststoff-Chips (kostengünstige Spritzguss-Verfahren) sowie andere Ausführungen möglich.

Die Biochips anderer Technologien sind ebenfalls-als Disposable für wenige Messungen ausgelegt. Hier spricht jedoch der aufgrund der aufwendigen Herstellung der Chips sehr hohe Preis meist gegen das Wegwerfen des Chips nach nur einer oder ein paar Messungen.

### 5.7 Flexibilität der Anwendung

Die schnelle und kostengünstige Produktion ermöglicht eine Vielfalt von individuellen Anwendungen, bei denen z.B. unter Berücksichtigung von Sequenz- und Gendatenbanken im Internet gezielt Oligonukleotid-Arrays synthetisiert werden.

Durch die Verwendung eines einzigen, vielfach gewundenen oder spiralförmigen Kanals könnte eine Hybridisierung im (langsamen) Durchfluss etabliert werden, die auch die Detektion von seltenen Ereignissen (z.B. selten exprimierte Gene) ermöglicht. Damit würde ein chromatographisches Prinzip in die DNA Array Technologie eingeführt werden.

Durch die Verwendung von Di-, Tri- oder längeren Oligonukleotiden als Synthesebausteinen ist eine weitere Reduktion der Herstellungszeiten erreichbar. Vor allem für einfachere Arrays können Syntheseeinheiten direkt beim Kunden zur Anwendung kommen und damit die Zusammensetzung des Arrays endgültig individualisieren.

Die große Flexibilität der Technologie ist auch im Hinblick auf die Erkenntnis von Bedeutung, dass sich die Gene einzelner Individuen sehr stark unterscheiden, so dass man keinen generellen Genkatalog für alle Spezies anlegen kann. Der Träger eröffnet hier die Möglichkeit, zum Beispiel in einem ersten Messzyklus die Basisdaten, wie sie im Internet - frei zugänglich oder nur spezifisch für den Systemkunden - bereitgestellt sind mit den individuellen Unterschieden eines Patienten abzugleichen und aus den Ergebnissen einen entsprechenden zweiten DNA-Array zu bilden, welcher die eigentlichen Tests auf das Individuum angepasst durchführt.

Die erfindungsgemäße Lösung kann auch für die Synthese von Peptidsequenzen in den Kanälen verwendet werden. Damit würden für eine Vielzahl von Anwendungen hoch komplexe und zugleich kostengünstige Peptidarrays bereitgestellt werden.

### 6. Überblick über einige Aspekte der Erfindung

### 6.1 Ausführungsvarianten des Trägers

Bei der Gestaltung ebenso wie bei der Fertigung der Träger gibt es eine Vielzahl von Ausführungsvarianten. Bei der Anordnung der Kanäle im Träger über der Fläche der Detektionseinheit ist die Verwendung nur eines Kanals ebenso denkbar wie die Anordnung einer Vielzahl an parallelen Kanälen. So sind auf einer Fläche von 25 x 37 mm fertigungstechnisch problemlos 500 Kanäle (Stand der Technik: 500 parallele Kapillaren mit einem Durchmesser von 900 nm) mit einer Länge von 37 mm und jeweils etwa 750 Reaktionsbereichen anzuordnen. Die gleiche Anzahl an Reaktionsbereichen (500 x 750 = 375.000) ließe sich auch in einem einzigen schlangenförmigen Kanal mit etwa 20 m Länge unterbringen.

Der Vorteil nur eines Kanals liegt in der Präsentation der Probe an allen Messpunkten des Arrays und ist daher für die Suche nach seltenen Bestandteilen besonders geeignet. Ein Vielzahl an parallelen Kanälen hat den Vorteil, dass sich die Produktionszeiten bei der Träger-Synthese durch das Multiplexen von Einsatzstoffen und Kanälen sowie alle Strömungsvorgänge minimieren lassen. Deshalb ist diese Kanalanordnung für die Träger-Synthese sowie alle Analysen mit einer ausreichenden Anzahl an Kopien jedes Analyten in der Probe zu bevorzugen.

Um beide Vorteile in einem Träger zu nutzen ist es möglich die Einsatzstoffe bei der Träger-Synthese mittels paralleler Fittings am Zugang zu den Kanälen einzubringen, obwohl der Kanal von der Probeneingabe an nur aus einem einzigen, langen Mikrokanal besteht. Dieser Effekt kann auch durch die Integration von Ventilen in den Träger oder die umgebenden Gerätekomponenten erfolgen. So hat die Firma Biacore fluidisch angesteuerte Ventile in einem zweiteiligen Spritzguss-Chip durch eine Membran realisiert, welche von unten in die Kanäle auf der Oberseite des Chips drückt und so die Kanäle verschließt.

Als Anordnung für die Kanäle über der Detektorfläche ist eine Vielzahl an Strukturen und Mikrokanalverläufen möglich. Für eine hohe Parallelität der fluidischen Vorgänge sind beispielsweise parallele oder "snakeförmige" Strukturen naheliegend. Die Aufteilung der Kanäle sollte hierbei nach dem Dualprinzip erfolgen, wo aus jedem Kanal zwei neue entstehen und alle Kanäle gleich lang sind. So erreicht man nach 10 Teilungen bereits 2¹⁰ = 2048 Kanäle. Spiralförmige Anordnungen haben den Vorteil, das sie weniger turbulente Strömungsvorgänge aufweisen und besser zu reinigen sind. Ihr großer Nachteil liegt in der Zu- bzw. Abführung, welche in der dritten Dimension nach oben oder unten erfolgen muss, was fertigungstechnisch und optisch eher ungünstig ist.

Als Material für die Träger ist beispielsweise Glas, Silizium, Keramik, Metall oder/und Kunststoff möglich. Der Aufbau kann in zwei Schichten erfolgen, welche zum Beispiel durch Kleben oder Bonden aneinandergefügt werden können oder nicht. Die Struktur der Kanäle kann hierbei entweder nur in die eine, oder aber in beide Seiten bzw. Hälften eingebracht werden. Hierfür sind als Fertigungsverfahren u.a. Laser oder Präzisionsfräsen verwendbar. Besonders kostengünstig ist Spritzguss, welcher in einer ausreichenden Qualität gefertigt werden kann. Weitere Verfahren sind die LIGA-Technik oder Heißformen.

### 6.2 Träger - Synthese

Für die Synthese der individuellen Fänger-Rezeptoren, z.B. Oligos, auf den Reaktionsbereichen im Träger-Array gibt es prinzipiell zwei Möglichkeiten. Der Kunde ersteht fertige Träger vom Hersteller mit einer vorgegebenen Auswahl an immobilisierten Basen- Sequenzen, oder er synthetisiert sich in einer Syntheseeinheit seine selbstgewählten Sequenzen auf ungelabelte Träger. Informationen über entsprechende Sequenzen können zum Beispiel aus Datenbanken im Internet entnommen werden, die hier frei oder auch speziell durch den Träger- Hersteller bereitgestellt werden.

### 6.2.1 Syntheseeinheit

Die Syntheseeinheit besteht aus einer geeigneten Lichtquelle, welche die Reaktionsbereiche im Träger-Array bei der Synthese der Rezeptoren, z.B. Basen oder Basen-Sequenzen, an der Trägeroberfläche bzw. den Kanalwänden ortsspezifisch hochgenau und exakt auflösend bestrahlt. Wie bereits unter 4.4 erwähnt, kann die Belichtung mittels einer programmierbaren Lichtquellenmatrix erfolgen. Verwendet werden kann auch eine Photolithographie- Einrichtung, wie sie in der Halbleiter- Chip-Produktion für das lichtaktivierte Ätzen von Si- Wafern zum Einsatz kommt.

### 6.2.2 Fertige Träger - Synthese beim Hersteller

Beim Vertrieb fertiger Träger erfolgt die Synthese beim Hersteller. Dieser benötigt hierfür eine entsprechend leistungsfähige Syntheseeinheit, welche möglichst lange Oligos (3 oder mehr Basen lang) als Einsatzstoffe verwendet, die parallel in die Kanäle eingebracht (eingespritzt) werden, und so die Synthesezeiten pro Träger minimieren (Multiplexing). Hierbei ist es möglich, in den Trägern spezielle Zugänge vorzusehen, mit dem Ziel möglichst viele parallele und damit kurze Kanäle zu erhalten, unabhängig davon, welche Kanalstruktur für den Analysevorgang vorgesehen ist.

### 6.2.3 Einsatzstoffe im Träger

Für Anwendungen, wo es nicht auf eine schnelle Synthese der Träger, aber auf eine individuelle-Gestaltung der Arrays ankommt, ist eine Bereitstellung der Einsatzstoffe (G, A, C, T und Puffer etc.) direkt im Träger in entsprechenden Reservoirs möglich. Die überflüssigen Einsatzstoffe müssen in einer entsprechenden Kammer im Träger aufgefangen werden. Das Volumen einer solchen Kammer ist durch eine Ausdehnung in der dritten Dimension nach oben oder unten problemlos auf ein Vielfaches des gesamten Kanalvolumens auslegbar. Eine Anwendungen dieser Träger-Variante ist gerade für Forschungslabors, aber auch kleine Arztpraxen denkbar.

Das Prinzip der Kapillarkraft kann hierbei in einer möglichen Ausführungsvariante direkt für den Fluidtransport im Träger verwendet werden. Jegliche Mechanik würde entfallen und die Befüllung der Kapillaren mit den Einsatzstoffen sowie der Probe könnte über die einfache Verstellung eines Ventils im Träger erfolgen. Die "Abfallkammer" könnte durch die Einbettung eines geeigneten Vlies-Stoffes eine unterstützende Saugwirkung entwickeln. Um eine Minimierung der benötigten Fluidmengen zu erreichen, sollte bei diesen Einwegströmungs- Ausführungen (keine Zirkulation und damit Wiederverwendung der Einsatzstoffe) auf immer gleich lange Kapillaren geachtet werden. Dies ist ebenfalls von Bedeutung für das Funktionieren der Kapillarkraft als Pumpe.

Ein weitere Variante ist eine vertikale Ausrichtung der planaren Träger, so dass auch Gravitationskräfte für den Fluidtransport im Träger genutzt werden können. Wenn diese Kräfte nicht ausreichen, um alle notwendige Fluidtransporte in den Träger zu realisieren, so sind andere geeignete Pumpmechanismen vorzusehen. Eine Möglichkeit hierzu ist eine elektrophoretische Bewegung der Fluide durch - in den Träger integrierte - Elektroden, oder durch eine Volumenreduktion in Kammern des Träger durch einen entsprechenden Krafteintrag von außen in den Träger (klassische Pumpe).

### 6.2.4 Einsatzstoffe in der Syntheseeinheit

Die Bereitstellung der Einsatzstoffe für die Träger-Synthese in Vorratsbehältern bietet prinzipiell den Vorteil des Multiplexens von fertigen Basen-Sequenzen und parallelen Kanälen, weshalb diese Ausführungsvariante für (Ultra)Hochdurchsatz-Screening und Träger-Hersteller empfehlenswert ist. Das Multiplexen kann an der Schnittstelle zum Träger erfolgen, in dem eine spezifische Basen- Sequenz für jeden Synthese-Zyklus einen anderen Kanal benetzt. Eine technisch aufwendigere, aber ggf. zuverlässigere Methode ist ein Multiplexen im Gerät durch ein entsprechendes Ventilsystem. Hier ist die Verschleppung zu beachten, welche durch die Verwendung unterschiedlicher Basen-Sequenzen entstehen kann.

Ein weiterer Punkt, welcher berücksichtigt werden muss, ist das Auffangen und Beseitigen des überschüssigen Materials am Ausgang der einzelnen Kanäle. Hier ist sowohl eine Zirkulation (Wiederverwendung des austretenden Materials) als auch eine Beseitigung der austretenden Einsatzstoffe denkbar.

### 6.3 Analytbestimmung

Die Analyse von Nukleinsäure- Sequenzen erfolgt wie bei anderen Oligonukleotid-Arrays durch Hybridisieren von Nukleinsäuren im Probenmaterial an komplementäre Stränge unter den immobilisierten Oligonukleotiden.

Als eine weitere mögliche Anwendung des Trägers können auch Peptidsequenzen in den Kanälen angekoppelt werden, ebenfalls nach in situ Syntheseprinzipien. Solche Peptide sind zu vielfältigen und teilweise hochspezifischen Bindungsreaktionen mit Peptiden, Proteinen und anderen Substanzen in der Lage, so dass sich das Spektrum potentieller Analyten erheblich ausweiten läßt.

Die Synthese im Träger würde erstmals massiv parallele und gleichzeitig kostengünstige Peptid-Arrays für eine Vielzahl von Anwendungen zur Verfügung stellen.

### 6.3.1 Analyten

Beispiele für Analyten sind Nukleinsäuren (DNA, RNA, in Spezialfällen auch PNA). Diese Nukleinsäuren können aus Gesamtgenomen, Fragmenten davon, Chromosomen, Plasmiden oder synthetischen Quellen (z.B. cDNA) gewonnen werden. In einer Ausführungsform kann das Probenmaterial aus dem menschlichen Genom stammen.

Weitere Beispiele für Analyten sind Proteine, Polypeptide und Peptide in allen Erscheinungsformen z.B. Hormone, Wachstumsfaktoren, Enzyme, Tumorantigene, Serumfaktoren, Antikörper, Carbohydrate, z.B. verschiedene Zucker in Lebensmitteln oder Agrarpflanzen, funktionelle Zucker, Polymere und andere organische Moleküle, z.B. drugs of abuse', Pharmaka, Metabolite, Aminosäuren, Transmitter, Pestizide, Insektizide, Lacke, verschiedene Toxine etc.

### 6.3.2 Varianten zur Bindung an den immobilisierten Interaktionspartner (Rezeptor)

Die Bindung des Analyten an den Rezeptor kann bei Nukleinsäuren durch Hybridisierung von komplementären Nukleinsäuren z.B. längere Moleküle wie cDNA, synthetische Oligonukleotide, PNA, RNA erfolgen. Peptide als Rezeptoren , z.B. synthetische Peptide oder natürliche Peptide können über Protein-Protein oder Protein-Nukleinsäure-Wechselwirkungen an den Analyten binden.

### 6.3.3 Varianten zur Signalerzeugung

Zwei Prinzipien zur Signalerzeugung werden bevorzugt eingesetz, nämlich: die direkte Detektion eines vorher oder in der Reaktion markierten Analyten (bevorzugte Methode in der Nukleinsäureanalytik mittels Hybridisierung) und die indirekte Detektion durch Kompetition des Analyten bzw. der Zielsequenz mit einem markierten Standard. Die erste Variante ist für einige Anwendungen gut etabliert, für Diagnostik z.B. von Serumkomponenten aber eher schlecht geeignet, die mit Peptid-Arrays auch im Träger möglich ist. Die zweite Variante ist für diese Anwendungen daher vorzuziehen, außerdem erlaubt sie prinzipiell eine einfachere Probenvorbereitung durch den Anwender.

Eine direkte Detektion kann erfolgen durch Markierung der Analyten mit einem Farbstoff für die Absorptionsmessung, einem Fluoreszenzfarbstoff, Markierung der Analyten mit Reporterenzym, anschließend Reaktion (z.B. Chemo- oder Biolumineszenz), selektive Markierung des gebundenen Analyten, z.B. bei Nukleinsäuren durch interkalierende (Fluoreszenz-) Farbstoffe, Doppelstrang-bindende Proteine oder Doppelstrang-bindende Antikörper oder eine sekundäre Detektion des gebundenen Analyten mit einer zweiten Komponente, z.B. bei PNA-DNA Hybriden durch DNA spezifischen Antikörper. Als markierte Standards verwendet werden können enzymgekoppelte Standards (z.B. Chemo- und Biolumineszenz mit alkalischer Phosphatase, Peroxidase etc.) oder Fluoreszenz-) Farbstoff gekoppelte Standards. Proteinstandards können als Fusionsproteine mit einem Reporterenzym (siehe oben) oder einem autofluoreszierendem Protein (z.B. GFP), z.B. für rekombinante Antikörper, Protein-Hormone, Wachstumsfaktoren etc. eingesetzt werden.

### 6.4 Bereitstellung des Probenmaterials

Für die Bereitstellung des Probenmaterials gibt es ebenfalls wieder unterschiedliche Ausführungsvarianten. Für die eigentliche Detektion ist die Art der Bereitstellung nicht relevant, da an der Schnittstelle immer in Flüssigkeit gelöste DNA-Fragmente in ausreichender Menge für die angestrebte Untersuchung bereitzustellen sind.

### 6.4.1 Externe Probenvorbereitung

Die Probenvorbereitung kann entweder manuell im Labor, in einem getrennten Analysesystem oder in einer in das gleiche System integrierten Vorbereitungseinheit erfolgen. Die detektionsbereite Probe wird dann mittels manuellem oder automatischem Pipettieren oder vergleichbaren Verfahren in den Träger eingebracht.

### 6.4.2 Probenvorbereitung im gleichen Träger "all in one"

Gerade, wenn das Multiplexen bei der Träger-Synthese zur Reduktion der Produktionszeiten verwendet wird, können gleiche oder sogar kürzere Zeiten für die Rezeptorsynthese erreicht werden, als z.B. für die DNA-Amplifizierung der Probe mittels PCR notwendig wäre. Dadurch wird eine Integration einer PCR in das Synthese- System oder sogar in den Träger für viele Anwendungen sinnvoll.

Neben der zeitintensiven PCR ist auch der vorgelagerte Zellaufschluss zum Beispiel über gut automatisierbare Verfahren wie Ultraschall oder Hochspannung ebenso wie die DNA-Isolierung integrierbar.

### 6.5 Detektionseinheit

Die Auslesung der Lichtsignale für die Nachweisreaktionen im Träger-Array soll in einer Detektionseinheit erfolgen, wobei die Anregungslichtquelle (Fluoreszenz, Lumineszenz oder Absorption als optischer Nachweis) dem CCD-Chip zur Lichtsignalmessung direkt gegenüber angeordnet wird. Der Träger-Array befindet sich zwischen Lichtquelle und Detektions-Chip (Sandwichbauweise). Als Anregungs-lichtquelle kann eine Belichtungsmatrix herangezogen werden. Die räumliche Anordnung dieser Einheit kann je nach Bedarf erfolgen (z.B. Nutzung der Gravitation für Strömungsvorgänge im Chip). Durch diese möglichst kompakte Bauweise werden die Lichtlaufwege und damit auch die benötigte Lichtintensität minimiert. Auf die Verwendung einer aufwendigen, platzintensiven, lichtschluckenden und teuren Optik soll sowohl auf der Anregungs-, als auch auf der Detektionsseite verzichtet werden.

### 6.5.1 Temperatur bei der Hybridisierung

Die Temperierung (derzeit typischerweise bei 60°C - neueste Entwicklungen ermöglichen auch schon eine Hybridisierung bei 25°C mit Niedrigsalz- Bedingungen) bei der Hybridisierung kann entweder durch entsprechende Temperaturelemente in der Detektionseinheit oder durch die Anregungslichtquelle bzw. das Anregungslicht an sich erfolgen. Temperaturelemente in den Trägern sind ebenfalls möglich.

### 6.5.2 Anregungslichtquelle

Als Lichtquellen kommen je nach den Markern der Analyten (Nachweisverfahren via Absorption oder Fluoreszenz etc.) hochparalleles Licht aus einer Lampe (Weißes Licht), hochparalleles Licht aus einer Blitzröhre, hochparalleles monochromatisches Licht, ein monochromatischer Laserlichtstrich, eine flächige Belichtung mittels Aufweitung des Laserstrahls, ein monochromatischer Laserstrahl oder eine programmierbare Lichtquellenmatrix in Frage.

Gegebenenfalls kann ein entsprechendes optisches Gitter oder eine entsprechende Optik zwischen Anregungslichtquelle und Träger-Array vorgesehen sein.

### 6.5.3 CCD-Kamera Detektion

Die Detektionseinheit besteht vorzugsweise nur aus einem CCD-Chip. Diese haben aktuell auf einer Fläche von beispielsweise 25 x 37 mm etwa 2000 x 3000 Pixel (Cannon). Ordnet man auf einer solchen Fläche von 25 x 37 mm etwa 500 parallele Kanälen mit ca. 20 µm Durchmesser an (jede zweite Doppel-Pixelreihe), so erhält man in jedem Kanal 750 Messpunkte (Felder), wenn man nur jeden zweiten Doppel-Pixel unter dem Kanal nutzt. Damit hätte man 375.000 Reaktionsbereiche auf einem einzigen Träger, wobei jeder Reaktionsbereich 4 Farb- bzw. 12 Schwarzweiß-Pixel überdeckt und eine Fläche von 20 x 20 µm hat. Die Lichtsignale müssen möglichst dicht am optischen CCD-Chip erzeugt werden, damit eine fehlerhafte Zuordnung von Lichtsignalen und Messpunkten mit ihrer spezifischen Basen- Sequenz sowie eine Überlagerung benachbarter Lichtsignale ausgeschlossen werden kann. Ansonsten kann eine serielle Detektion sich überlagernder Bereiche erfolgen oder es werden faseroptische Elemente eingesetzt.

Die entstehende Vielzahl an Messwerten (4 x 500 x 750 = 1.5 Mio. Farbsignale bzw. 4.5 Mio. Intensitätswerte zwischen 0 und 4096 Digitalwerten), welche zur Verfügung stehen (aktueller Stand der CCD-Chip Technik), bilden die Basis welche eine umfangreiche Statistik bei der Analyse der detektierten Lichtsignale erlaubt. Das Bearbeiten der anfallenden Datenmengen ist, durch die Entwicklung der Leistungsfähigkeit bei gleichzeitigem Preisverfall von modernen Rechnersystemen, problemlos möglich.

Die- Detektion der Nachweisreaktion kann sowohl qualitative als auch quantitative Aussagen machen und zwar welche Fängermoleküle (Position im Array) haben Anlagerungspartner gefunden (Auswertung der z.B. fluoreszenzmarkierten Labels) und wieviele Fängermoleküle einer Klasse haben einen Hybridisierungspartner gefunden.

Gegebenenfalls kann ein entsprechendes optisches Gitter oder eine entsprechende Optik zwischen dem Träger-Array und dem CCD-Kamera Chip vorgesehen sein.

Wenn die Detektion mit einer CCD-Kamera bzw. einem CCD-Chip keine ausreichenden Signale ergibt, kann die Detektion im Analysesystem auch mittels anderer, empfindlicherer Sensoren erfolgen.

Interessant im Zusammenhang mit der vorliegenden Erfindung ist die Verwendung einer Inspektionseinheit, wie in der deutschen Patentanmeldung 198 39254.0 beschrieben ist. Diese Inspektionseinheit umfasst eine elektronisch steuerbare Lichtquellenmatrix und eine der Lichtquellenmatrix zugewandt-gegenüberliegende Lichtsensormatrix, nämlich CCD-Bildaufnehmer.

In diesem Zusammenhang ist es denkbar, dass der Anwender sich seine Träger selbst erzeugt und direkt verwendet. Er lädt sich einfach die benötigten Daten (DNA-Sequenzen) von einer CD-ROM oder aus dem Internet und erzeugt in seiner Belichtungsmatrix-CCD-Einheit seinen individuellen DNA-Chip, benetzt ihn anschließend mit der Probe und liest die Signale aus.

Misst man z.B. jeden zweiten Pixel in dieser Anordnung für die Photoaktivterung, so kann man die Pixel dazwischen, welche in Projektion innerhalb eines Kanals liegen, für eine permanente Prozesskontrolle verwenden. So kann man z.B. das Einströmen einer Gasblase zwischen zwei Fluiden in einem Kanal individuell und dynamisch verfolgen. Auch ein Färben der Trägerfluide für G, A, C und T wäre denkbar, so dass die Anwesenheit der richtigen Oligos überprüfbar würde und eine Farbveränderung könnte eine Verschleppung signalisieren. Bei der anschließenden Detektion könnte wiederum eine ortsspezifische und wenn notwendig sogarfarbspezifische Lichtanregung erfolgen. Hierdurch ergeben sich ganz neue Möglichkeiten für Nachweisverfahren, wie sie derzeit noch nicht vorhanden sind.

Durch die Inspektionseinheit (Belichtungsmatrix-CCD-Einheit) können die Strömungsvorgänge in den Kanälen in einem Träger sowohl während der Produktion - sprich der Oligo-Synthese - als auch während der Analyse überwacht werden. Hierzu können z.B. Reinigungsgasblasen zwischen zwei Fluiden in den Kanälen oder eine Färbung der einzelnen Fluide verwendet werden.

Für die lichtinduzierte Abspaltung von Schutzgruppen während der Synthese von DNA-Oligos auf oder in dem Träger kann eine Belichtungsmatrix dienen, die die notwendige Wellenlänge von z.B. 360-370 nm erzeugt und überträgt.

Die Detektion der Nachweisreaktion im Träger kann ebenfalls in der Inspektionseinheit erfolgen. Wenn der Nachweis über Fluoreszenzmarker realisiert wird, müsste hierzu ggf. die Hintergrundbeleuchtung gewechselt werden (automatisch möglich), wobei optische Filter oder/und Glasfaserelemente ("Taper") verwendet werden können. Gegebenenfalls kommen hier auch neue Detektionsverfahren zum Einsatz, welche erst durch die extrem flexible, individuelle Anstrahlung und Detektion des einzelnen Reaktionsbreichs möglich-werden.

Für eine Standard-Hybridisierung von DNA-, RNA- und PNA-Strängen miteinander benötigt man eine Temperatur von ca. 55 - 65 °C. Im einfachsten Fall kann diese Temperatur durch die abgestrahlte Energie der Belichtungsmatrix erzeugt werden (Abwärme und Wellenlänge). Damit ließe sich eine weitere Kompaktierung der Anordnung erreichen.

### 8. Exemplarische Ausführungsformen

Die Synthese von DNA-Molekülen in Kanälen kann unter Verwendung von Standard-Synthonen, z.B. Phosphoramidit-Bausteinen, mit geeigneten Schutzgruppen, z.B. Dimethoxymethyl (DMT) erfolgen. Ausgehend von einem an die Festphase gekoppelten Linker kann eine entsprechende fluidische DNA-Synthese erfolgen.

Dieses Format kann für die bevorzugte Ausführungsform der Erfindung mit einer lichtabhängigen Steuerung der DNA-Synthese kombiniert werden. Dazu sind Schutzgruppen bekannt, die eine lichtabhängige Entschützung erlauben, so dass die Schutzgruppe, die meist am 5'-Kohlenstoffatom des Synthons gebunden ist, durch Licht geeigneter Wellenlänge abgespalten wird. Auf diese Weise ist in Kapillaren die Synthese von Nukleinsäuren mit einer Länge von 18 oder mehr Nukleotiden möglich.

Durch Ablösung des synthetisierten DNA-Oligomere, wie es durch Verwendung geeigneter Linker möglich ist, können die Reaktionsprodukte z.B. durch Hochleistungflüssigchromatographie (HPLC) analysiert werden. Dabei lässt sich über den Anteil der Vollängenprodukte die Effizienz der kapillaren DNA-Synthese zeigen.

Für die lichtabhängige DNA-Synthese wird der Reaktionsbereich auf dem Träger orts- oder/und zeitspezifisch mit einer geeigneten Lichtquelle belichtet, z.B. mit einer Quecksilberdampflampe, Laserlicht (z.B. Stickstofflaser mit 373 nm) oder mit einer UV-LED. Ebenso geeignet sind auch andere Lichtquellen, die eine ausreichend energiereiche Strahlung aufweisen.
- Fig. 1: zeigt in einer stark schematisierten Draufsicht einen Träger nach der Erfindung.
- Fig. 2: zeigt Beispiele für Kanalanordnungen in einem Träger nach der Erfindung.
- Fig. 3: zeigt eine schematische Darstellung eines Trägers in einer lnspektionseinheit aus programmierbarer Lichtquellenmatrix und CCD-Matrix.
- Fig. 4: zeigt eine schematische Darstellung einer erfindungsgemäßen Vorrichtung für ein lichtgestütztes integriertes Synthese- und Analyseverfahren und
- Fig. 5: zeigt den Aufbau aus Fig. 4 für eine fluidische Individualisierung von Reaktionsbereichen.

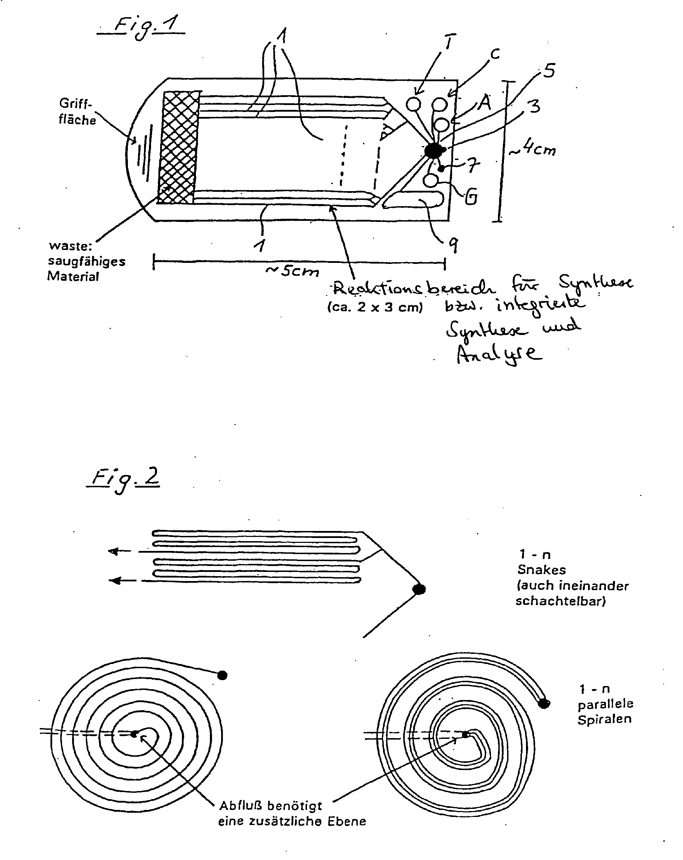

Figur 1 zeigt einen den transparenten Träger in einer Draufsicht in stark schematisierter Weise. Man erkennt die parallel zueinander verlaufenden Kanäle 1, beispielsweise 500 Kanäle mit einer Länge von 37 nm. Mit T, G, A, C sind in Fig. 1 Reservoirs für die einzelnen Einsatzstoffe (Basen) bezeichnet. Mit 3 ist der Gaseinlass bezeichnet. 5 kennzeichnet ein Ventil. 7 kennzeichnet die Probeneingabe und mit 9 ist ein Zugang für weitere Synthesechemikalien sowie Reinigungs-/Waschflüssigkeit bezeichnet.

In Figur 2 sind schematisch weitere Beispiele für alternative Kanatanordnungen dargestellt.

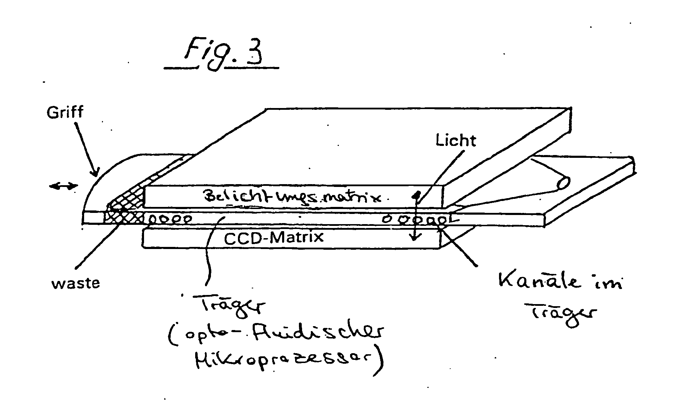

Figur 3 zeigt den Träger nach Fig. 1 in einer Inspektionseinheit aus programmierbarer Lichtquellenmatrix, z.B. einer LCD-Matrix und einer CCD-Detektionsmatrix.

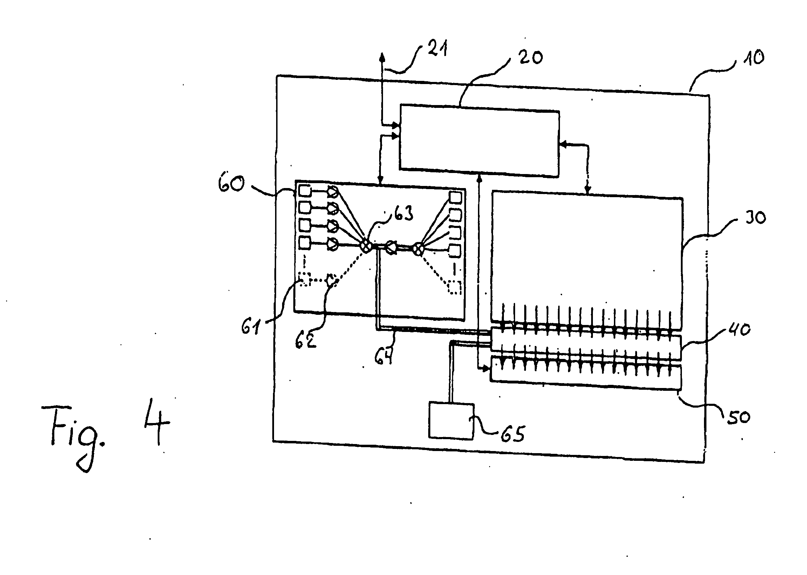

In Figur 4 ist eine erfindungsgemäße Vorrichtung mit einem auswechselbaren Träger 40 dargestellt, wobei der prinzipielle Aufbau unabhängig davon ist, ob der Träger in jedem Zyklus oder erst bei Verschleiß ausgewechselt wird. In letzterem Fall findet eine Reinigung und anschließende Wiederverwendung derselben Kanäle statt. Dargestellt ist eine programmierbare Lichtquellenmatrix 30. Deren Programmierbarkeit kann in die Systemkomponente 20, die aus einem Rechner bzw. einem Computer besteht, integriert sein, so dass nur eine frei ansteuerbare Lichtquellenmatrix als Komponente 30 notwendig ist. Diese Lichtquellenmatrix 30 strahlt Licht definierter Wellenlänge und Intensität auf beliebig ansteuerbare Orte einer zumindest zweidimensionalen Matrix, die der hochparallelen Belichtung der Reaktionsbereiche im Träger 40 dient. Besagter Träger 40 wird durch die Lichtquellenmatrix 30 mit dem rechnergesteuerten Lichtmuster bestehend aus Energiewellen in allen Reaktionsbreichen individuell bestrahlt. Über das fluidische Anschlusssystem 64 werden die vom Fluidikmodul 60 bereitgestellten Fluide in den Träger 40 transportiert und in dessen in der Zeichnung nicht dargestellten Mikrostruktur in geeigneter Weise an die Reaktionsbereiche weitergeleitet. Dadurch wird der Träger 40 zu einem opto-fluidischen Mikroporzessor. Dieser kann entweder nach jeder Anwendung gewechselt werden oder nach jeder Anwendung gereinigt und nur zu Servicezwecken bei Verschleiß gewechselt werden.
Das eintretende Licht kann zum Beispiel für Absorptionsmessungen, die Aktivierung von Photoreaktionen oder das Anregen von Fluoreszenz genutzt werden.

Das aus dem Träger 40, bzw. dem opto-fluidischen Mikroprozessor austretende Licht kann beispielsweise das im Durchlicht den Träger passierende Licht der Lichtquellenmatrix- 30 sein. Es kann sich dabei jedoch auch um Lichtsignale handeln, die in den einzelnen Reaktionsbereichen des Trägers 40 durch beispielsweise Fluoreszenz oder Lumineszenz erzeugt werden.

Die Detektormatrix 50, die zum Beispiel aus einem CCD-Chip mit oder ohne Optik besteht, ist so gegenüber einer Lichtquellenmatrix 30 mit einem dazwischen liegenden Träger 40 angeordnet, dass dadurch eine dreifache Matrixanordnung aus Licht-, Träger und Detektormatrix entsteht.

Das Fluidmodul 60 dient der Versorgung des Reaktionsträgers 40 zum Beispiel mit Einsatzstoffen, Schutzgasen, Chemikalien, wie Lösemitteln, etc. und Probenmaterial. Das Fluidmodul 60 besteht aus Tanks 61, die durch Pumpen 62 und Ventile 63 in geeigneter Weise entleert werden. Die Tanks können einzeln oder im Cluster ausgewechselt oder neu gefüllt werden. Permanent benötigte Fluide, wie beispielsweise Schutzgas, können auch mittels Leitungen kontinuierlich (ohe Tanks im System) zugeführt werden. Der fluidische Abfall der verschiedenen Verfahren kann entweder im Träger 40 in integrierten Tanks oder in einem Abfallsystem 65 oder bei Clustern außerhalb des einzelnen Systems aufgefangen werden.

Ebenfalls dargestellt ist die Systemgrenze 10 der Vorrichtung, die als Einzelgerät oder auch in zentralen oder dezentralen Clustern eingesetzt werden kann. Diese Cluster sind immer informationstechnisch miteinander verknüpft. Die an einem Ort befindlichen Systeme können auch gemeinsam durch manuelle Bedienung oder automatisierte Komponenten mit Energie, Fluiden, wie Einsatzstoffen, Reaktionschemikalien, Schutzgasen und Probenmaterial, sowie mit den benötigen Trägern versorgt werden.

Die Systemkomponente 20 in Form eines Computers oder Rechners übernimmt die Steuerung bzw. Regelung des Systems. Hierunter fallen auf der Basis der Berechnung der Sonden- bzw. Rezeptorsequenzen für die einzelnen Reaktionsbereiche die Steuerung der Lichtquellenmatrix 30 sowie der Fluidkompnente 60. Ferner werden die Daten der De-ktormatrix 50 erfasst und ausgewertet.

Jede Vorrichtung kann somit über seine Systemgrenze 10 hinweg mit anderen Vorrichtungen oder Systemen, bestehend aus wiederum einer erfindungsgemäßen Vorrichtung oder anderen Rechnern oder Datenbanken, kommunizieren. Dies kann beispielsweise über Leitungen, Bussysteme oder über das Internet erfolgen. Dabei kann die Kommunikation zentral koordiniert über Leitrechner erfolgen oder als Cluster gleichberechtigter Systeme. Ebenfalls vorgesehen ist eine Datenschnittstelle 21 zur Systemumgebung.

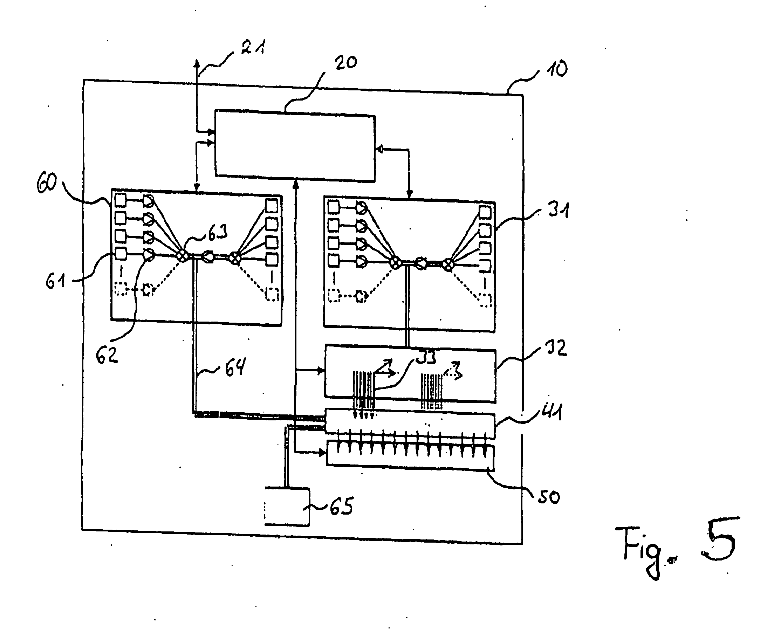

Figur 5 zeigt den Aufbau aus Figur 4 für eine fluidische Individualisierung der Reaktionsbereiche. Wiederum dargestellt ist ein Träger 41. Dieser wird durch das fluidische Entschützungsmodul 32 rechnergesteuert individuell benutzt. Über das fluidische Anschlusssystem 64 werden die vom Fluidmodul 60 bereitgestellten Fluide in den Träger transportiert und in dessen in der Zeichnung nicht dargestellten Mikrostruktur in geeigneter Weise an die Reaktionsbereiche weitergeleitet. Dadurch wird der Träger 41 zu einem opto-fluidischen Mikroprozessor. Dieser kann entweder nach jeder Anwendung gewechselt werden oder nach jeder Anwendung gereinigt und nur zu Servicezwecken bei Verschleiß gewechselt werden.

In diesen Träger kann zum Beispiel von oben oder/und von der Seite Licht für die Anregung von Fluoreszenzreaktionen etc. eingespeist werden.

Das aus dem Träger, bzw. dem opto-fluidischen Mikroprozessor austretende Licht kann beispielsweise durch Lumineszenz an den Reaktionsbereichen erzeugt werden.

Das fluidische Entschützungsmodul32 kann jeden Reaktionsbereich auf dem Träger 41 mit mindestens einer der Benetzungskomponenten 33 (z.B. Düsen, Kapillaren, etc.) individuell mit Fluiden in Kontakt bringen. Hierdurch können zum Beispiel lokal chemische und biochemische Reaktionen aktiviert werden.

Das Fluidmodul 31 dient der Versorgung des fluidischen Entschützungsmoduls 32 mit Einsatzstoffen oder Chemikalien. Das Fluidmodul 31 ist dem Modul 60 vergleichbar aufgebaut und besteht je nach Bedarf aus Tanks, Leitungen, Ventilen, etc.

Die Detektormatrix 50, die zum Beispiel aus einem CCD-Chip mit oder ohne Optik besteht, ist so gegenüber einem fluidischen Entschützungsmodul 32 mit einem dazwischen liegenden Träger 41 angeordnet, dass dadurch wiederum eine dreifache Matrixanordnung entsteht.

Das Fluidmodul 60 dient der Versorgung des Trägers 41 zum Beispiel mit Einsatzstoffen, Schutzgasen, Chemikalien, wie Lösemitteln, etc. und Probenmaterial. Das Fluidmodul 60 besteht aus Tanks 61, die durch Pumpen 62 und Ventile 63 in geeigneter Weise entleert werden. Die Tanks können einzeln oder im Cluster ausgewechselt oder neu gefüllt werden. Permanent benötigte Fluide, wie beispielsweise Schutzgas, können auch mittels Leitungen kontinuierlich (ohne Tanks im Ssystem) zugeführt werden. Der fluidische Abfall der verschiedenen Verfahren kann entweder im Träger 41 in integrierten Tanks oder in einem Abfallsystem 65 oder bei Clustern außerhalb des einzelnen Systems aufgefangen werden.

Wiederum dargestellt ist die bereits erläuterte Systemgrenze 10 der Vorrichtung und die Systemkomponente 20 in Form eines Computers oder Rechners, der die Steuerung- bzw. Regelung des System übernimmt. Hierunter fallen auf der Basis der Berechnung der Sondensequenzen für die einzelnen Reaktionsbereiche die Steuerung der Fluidmodule 31 und 60, sowie des fluidischen Entschützungsmoduls 32. Ferner werden die Daten der Detektormatrix 50 erfasst und ausgewertet.

Jede Vorrichtung kann somit über seine Systemgrenze 10 hinweg mit anderen Vorrichtungen oder Systemen, bestehend aus wiederum einer erfindungsgemäßen Vorrichtung oder anderen Rechnern oder Datenbanken, kommunizieren. Dies kann beispielsweise über Leitungen, Bussysteme oder über das Internet erfolgen. Dabei kann die Kommunikation zentral koordiniert über Leitrechner erfolgen ode als Cluster gleichberechtigter Systeme. Ebenfalls vorgesehen ist eine Datenschnittstelle 21 zur Systemumgebung.

Die im Folgenden geeigneten Ausführungsformen stellen besonders bevorzugte Aspekte der Beschreibung dar.
14. Verfahren zur integrierten Synthese und Analytbestimmung an einem Träger umfassend die Schritte:
   (a) Bereitstellen eines Trägerkörpers,
   (b) Leiten einer Flüssigkeit mit darin enthaltenen Rezeptoren oder Bausteinen für die Synthese von polymeren Rezeptoren über den Träger,
   (c) orts- oder/und zeitspezifisches Immobilisieren der Rezeptoren oder Rezeptorbausteine an jeweils vorbestimmten Positionen auf dem Träger,
   (d) gegebenenfalls Wiederholen der Schritte (b) und (c) bis die gewünschten Rezeptoren an den jeweils vobestimmten Positionen auf dem Träger synthetisiert worden sind,
   (e) Inkontaktbringen des Trägers mit einer Analyten enthaltenden Probe und
   (f) Bestimmen der Analyten über deren Bindung an die auf dem Träger immobilisierten Rezeptoren.
15. Verfahren nach Punkt 14,
   dadurch gekennzeichnet,
   dass die Synthese und Analytbestimmung in einer integrierten Vorrichtung durchgeführt wird, wobei der Synthese- oder/und der Analytbestimmungsprozess in einer beliebigen Anzahl von Positionen auf dem Träger überwacht und geregelt wird.
16. Verfahren nach Anspruch 15,
   dadurch gekennzeichnet,
   dass man eine integrierte Vorrichtung verwendet, umfassend eine programmierbare Lichtquellenmatrix, eine Detektormatrix, einen zwischen Lichtquellen - und Detektormatrix angeordneten Träger sowie Mittel zur Zufuhr von Fluids in den Träger und zur Ableitung von Fluids aus dem Träger.
17. Verfahren nach einem der Punkte 14 bis 16,
   dadurch gekennzeichnet,
   dass nach der Bestimmung der Analyt wieder vom Träger entfernt wird.
18. Verfahren nach einem der Punkte 14 bis 17,
   dadurch gekennzeichnet,
   dass mehrere Synthese/Analytbestimmungszyklen durchgeführt werden, wobei die Rezeptoren für einen nachfolgenden Zyklus auf Basis der Informationen aus einem vorhergehenden Zyklus synthetisiert werden.
19. Verfahren nach Punkt 18,
   dadurch gekennzeichnet,
   dass für den nachfolgenden Zyklus einer Verlängerung der Rezeptoren aus dem vorhergehenden Zyklus erfolgt.
20. Verfahren nach Punkt 18,
   dadurch gekennzeichnet,
   dass für den nachfolgenden Zyklus ein neuer Träger mit gegenüber dem vorhergehenden Zyklus modifizierten Rezeptoren synthetisiert wird.
21. Verfahren nach Punkt 20,
   dadurch gekennzeichnet,
   dass die Modifizierung der Rezeptoren eine Änderung der Sequenz oder/und einen Ausschluss negativer Rezeptoren des vorhergehenden Zyklus umfasst.
22. Verfahren nach einem der Punkte 14 bis 21,
   dadurch gekennzeichnet,
   dass man einen planaren Träger verwendet.
23. Verfahren nach einem der Punkte 14 bis 21,**dadurch gekennzeichnet,**
   dass man einen Träger mit einer Vielzahl von Kanälen verwendet.
24. Verfahren nach einem der Punkte 14 bis 23,
   dadurch gekennzeichnet,
   dass für einen Synthese/Analytbestimmungszyklus mehrere Träger verwendet werden.
25. Verfahren nach Punkt 24,
   **dadurch gekennzeichnet,**
   dass die mehreren Träger in unterschiedlichen Detektionsvorrichtungen synthetisiert und analysiert werden, die informationstechnisch miteinander verknüpft sind, jedoch voneinander räumlich getrennt sein können.
30. Vorrichtung nach Punkt 29 weiterhin umfassend Mittel zur Entschützung von Reaktionskomponenten auf den Träger.
31. Vorrichtung nach Punkt 29 oder 30 weiterhin umfassend elektronische Steuerungs- und Regelungsmittel.
32. Verwendung des Verfahrens nach einem der Punkte 1 bis 25, des Trägers nach Punkt 26 oder 27, des Reagenzienkits nach Punkt 28 oder der Vorrichtung nach einem der Punkte 29 bis 31 zur Bestimmung eines Analyten in einer Probe.
33. Verwendung nach Punkt 32 zur Sequenzierung von Nukleinsäuren.
34. Verwendung nach Punkt 33 zur Neusequenzierung oder/und Resequenzierung von komplexen genetischem Material, wie etwa individueller Genome oder synthetischen Nukleinsäuren.
35. Verwendung nach Punkt 32 zur Gewinnung diagnostischer Informationen für die individuelle Patientenversorgung wie etwa die individuelle Wirkung von Pharmaka.
36. Verwendung nach Punkt 32 zur Wirkungsanalyse pharmakologischer Substanzen.
37. Verwendung nach Punkt 32 zur Erstellung und Analyse von Substanzbibliotheken.
38. Verwendung nach Punkt 32 zum Vergleich von Individiuen in einer Population.

## Patentansprüche

1. Verfahren zur integrierten Synthese und Analytbestimmung an einem Träger umfassend die Schritte:
(a) Bereitstellen eines Trägerkörpers,
(b) Leiten einer Flüssigkeit mit darin enthaltenen Rezeptoren oder Bausteinen für die Synthese von polymeren Rezeptoren über den Träger,
(c) orts- oder/und zeitspezifisches Immobilisieren der Rezeptoren oder Rezeptorbausteine an jeweils vorbestimmten Positionen auf dem Träger,
(d) gegebenenfalls Wiederholen der Schritte (b) und (c) bis die gewünschten Rezeptoren an den jeweils vorbestimmten Positionen auf dem Träger synthetisiert worden sind,
(e) Inkontaktbringen des Trägers mit einer Analyten enthaltenden Probe und
(f) Bestimmen der Analyten über deren Bindung an die auf dem Träger immobilisierten Rezeptoren,
**dadurch gekennzeichnet, dass** in einem Synthese/ Analytbestimmungszyklus mehrere Träger synthetisiert werden und eine direkte logische Verknüpfung zwischen den Ergebnissen der Analyse eines ersten Trägers und der Synthese des darauf folgend zu synthetisierenden Trägers erfolgt, wobei die Rezeptoren für einen nachfolgenden Träger auf Basis der Informationen aus dem vorhergehenden Träger synthetisiert werden.

2. Verfahren nach Anspruch 1,
**dadurch gekennzeichnet,**
**dass** die Rezeptoren ausgewählt werden aus Nukleinsäuren und Nukleinsäureanaloga, und das Verfahren eine zyklische Abfolge von Synthese, Sequenzvergleich, Analyse der Vergleichsergebnisse und wiederum Synthese von Rezeptoren am Träger umfasst.

3. Verfahren nach Anspruch 1 oder 2, wobei der Verlauf der Rezeptorsynthese mittels geeigneter Detektionseinrichtungen verfolgt und gesteuert wird.

4. Verfahren nach einem der Ansprüche 1 bis 3,
**dadurch gekennzeichnet,**
**dass** die Synthese und Analytbestimmung in einer integrierten Vorrichtung durchgeführt wird, wobei der Synthese- oder/und der Analytbestimmungsprozess in einer beliebigen Anzahl von Positionen auf dem Träger überwacht und geregelt wird.

5. Verfahren nach Anspruch 4,
**dadurch gekennzeichnet,**
**dass** man eine integrierte Vorrichtung verwendet, umfassend eine programmierbare Lichtquellenmatrix, eine Detektormatrix, einen zwischen Lichtquellen - und Detektormatrix angeordneten Träger sowie Mittel zur Zufuhr von Fluids in den Träger und zur Ableitung von Fluids aus dem Träger.

6. Verfahren nach Anspruch 5, wobei die Vorrichtung weiterhin Mittel zur Entschützung von Reaktionskomponenten auf den Träger umfasst.

7. Verfahren nach Anspruch 5 oder 6, wobei die Vorrichtung weiterhin elektronische Steuerungs- und Regelungsmittel umfasst.

8. Verfahren nach einem der Ansprüche 1 bis 7,
**dadurch gekennzeichnet,**
**dass** nach der Bestimmung der Analyt wieder vom Träger entfernt wird.

9. Verfahren nach einem der Ansprüche 1 bis 8,
**dadurch gekennzeichnet,**
**dass** der darauf folgend synthetisierte Träger mit gegenüber dem ersten Träger verlängerten Rezeptoren synthetisiert wird.

10. Verfahren nach einem der Ansprüche 1 bis 8,
**dadurch gekennzeichnet,**
**dass** der darauf folgend synthetisierte Träger mit gegenüber dem ersten Träger modifizierten Rezeptoren synthetisiert wird.

11. Verfahren nach Anspruch 10,
**dadurch gekennzeichnet,**
**dass** die Modifizierung der Rezeptoren eine Änderung der Sequenz oder/und einen Ausschluss negativer Rezeptoren des vorhergehenden Zyklus umfasst.

12. Verfahren nach einem der Ansprüche 1 bis 11,
**dadurch gekennzeichnet,**
**dass** man einen planaren Träger verwendet.

13. Verfahren nach einem der Ansprüche 1 bis 12,
**dadurch gekennzeichnet,**
**dass** man einen Träger mit einer Vielzahl von Kanälen verwendet.

14. Verfahren nach einem der Ansprüche 1 bis 13,
**dadurch gekennzeichnet,**
**dass** die mehreren Träger in unterschiedlichen Detektionsvorrichtungen synthetisiert und analysiert werden, die informationstechnisch miteinander verknüpft sind, jedoch voneinander räumlich getrennt sein können.

15. Verwendung des Verfahrens nach einem der Ansprüche 1 bis 14 zur Bestimmung eines Analyten in einer Probe.

16. Verwendung nach Anspruch 15 zur Sequenzierung von Nukleinsäuren.

17. Verwendung nach Anspruch 16 zur Neusequenzierung oder/und Resequenzierung von komplexen genetischern Material, wie etwa individueller Genome oder synthetischen Nukleinsäuren.

18. Verwendung nach Anspruch 15 zur Gewinnung diagnostischer Informationen für die individuelle Patientenversorgung wie etwa die individuelle Wirkung von Pharmaka.

19. Verwendung nach Anspruch 15 zur Wirkungsanalyse pharmakologischer Substanzen.

20. Verwendung nach Anspruch 15 zur Erstellung und Analyse von Substanzbibliotheken.

21. Verwendung nach Anspruch 15 zum Vergleich von individuen in einer Population.

## Claims

1. Method for the integrated synthesis and analyte determination on a support, comprising the steps:
(a) providing a support body,
(b) passing a liquid with receptors contained therein or building blocks for the synthesis of polymeric receptors contained therein over the support,
(c) site- or/and time-specifically immobilising the receptors or receptor building blocks on in each case predetermined positions on the support,
(d) if necessary repeating steps (b) and (c) until the required receptors have been synthesised on the in each case predetermined positions on the support,
(e) contacting the support with an analyte-containing sample,
(f) determining the analytes via their binding to the receptors immobilised on the support,
**characterised in that** several supports are synthesised in one synthesis/analyte determination cycle and a direct logical link occurs between the results of the analysis of a first support and the synthesis of the support to be synthesised next, wherein the receptors for the next support are synthesised based on the information from the previous support.

2. Method according to claim 1, **characterised in that** the receptors are selected from the group consisting of nucleic acids and nucleic acid analogues, and **in that** the method comprises a cyclic succession of synthesis, sequence comparison, analysis of comparison results and again synthesis of receptors on the support.

3. Method according to claim 1 or 2, wherein the course of the receptor synthesis is traced and operated via appropriate detection appliances.

4. Method according to any one of claims 1 to 3, **characterised in that** the synthesis and analyte determination is carried out in an integrated device, wherein the synthesis- or/and the analyte determination-process is monitored and controlled at any number of positions on the support.

5. Method according to claim 4, **characterised in that** one uses an integrated device, comprising a programmable light source matrix, a detector matrix, a support arranged between light source matrix and detector matrix, and means for feeding of fluids into the support and for the discharging of fluids from the support.

6. Method according to claim 5, wherein the device further comprises means for the deprotection of reaction components on the support.

7. Method according to claim 5 or 6, wherein the device further comprises electronic operation and control means.

8. Method according to any one of claims 1 to 7, **characterised in that** after the determination, the analyte is removed from the support.

9. Method according to any one of claims 1 to 8, **characterised in that** the support synthesised next is synthesised with prolonged receptors compared to the first support.

10. Method according to any one of claims 1 to 8, **characterised in that** the support synthesised next is synthesised with modified receptors compared to the first support.

11. Method according to claim 10, **characterised in that** the modification of the receptors comprises an alteration of the sequence or/and an exclusion of negative receptors of the previous cycle.

12. Method according to any one of claims 1 to 11, **characterised in that** one uses a planar support.

13. Method according to any one of claims 1 to 12, **characterised in that** one uses a support with a plurality of channels.

14. Method according to any one of claims 1 to 13, **characterised in that** the several supports are synthesised and analysed in different detection appliances, which are information technologically linked to each other, but can be separated from each other spatially.

15. Use of the method according to any one of claims 1 to 14 for the determination of an analyte in a sample.

16. Use according to claim 15 for the sequencing of nucleic acids.

17. Use according to claim 16 for newly sequencing or/and re-sequencing of complex genetic material, as for example individual genomes or synthetic nucleic acids.

18. Use according to claim 15 for obtaining diagnostic information for individual patient care, as for example the individual effect of pharmaceuticals.

19. Use according to claim 15 for the analysis of the effects of pharmacological substances.

20. Use according to claim 15 for the building and analysis of substance libraries.

21. Use according to claim 15 for the comparison of individuals in a population.

## Revendications

1. Procédé pour la synthèse intégrée et la détermination d'analytes sur un support, comprenant les étapes consistant à:
(a) préparer un corps de support,
(b) envoyer un liquide contenant des récepteurs ou des éléments structuraux pour la synthèse de récepteurs polymères sur le support,
(c) immobiliser de manière spécifique vis-à-vis du lieu et/ou du temps les récepteurs ou les éléments de récepteurs dans des positions respectives prédéterminées sur le support,
(d) éventuellement répéter les étapes (b) et (c) jusqu'à ce que les récepteurs souhaités aient été synthétisés dans les positions respectives prédéterminées sur le support,
(e) mettre le support en contact avec un échantillon contenant des analytes, et
(f) déterminer les analytes par l'intermédiaire de leur liaison aux récepteurs immobilisés sur le support,
**caractérisé en ce que**, dans un cycle de synthèse/détermination d'analytes, on synthétise plusieurs supports et on établit une relation logique directe entre les résultats de l'analyse d'un premier support et la synthèse du support à synthétiser à la suite, les récepteurs pour un support suivant étant alors synthétisés sur la base des informations provenant du support précédent.

2. Procédé selon la revendication 1,
**caractérisé en ce que**
les récepteurs sont choisis parmi des acides nucléiques et des analogues d'acides nucléiques, et le procédé comprend une succession de cycles de synthèse, comparaison de séquences, analyse des résultats de la comparaison et à nouveau synthèse de récepteurs sur le support.

3. Procédé selon la revendication 1 ou 2, dans lequel le déroulement de la synthèse de récepteurs s'effectue et est commandé au moyen d'installations de détection appropriées.

4. Procédé selon l'une des revendications 1 à 3,
**caractérisé en ce que**
la synthèse et détermination d'analytes s'effectue dans un dispositif intégré, le processus de synthèse et/ou de détermination d'analytes étant contrôlé et réglé dans un nombre quelconque de positions sur le support.

5. Procédé selon la revendication 4,
**caractérisé en ce que**
l'on utilise un dispositif intégré, comportant une matrice de sources lumineuses programmable, une matrice de détecteurs, un support disposé entre la matrice de sources lumineuses et la matrice de détecteurs, ainsi que des moyens pour l'apport de fluide dans le support et pour l'évacuation de fluide hors du support.

6. Procédé selon la revendication 5, dans lequel le dispositif comprend en outre des moyens pour déprotéger les composants de réaction sur le support.

7. Procédé selon la revendication 5 ou 6, dans lequel le dispositif comprend en outre des moyens de commande et de réglage électroniques.

8. Procédé selon l'une des revendications 1 à 7,
**caractérisé en ce que**,
après la détermination, on sépare à nouveau l'analyte du support.

9. Procédé selon l'une des revendications 1 à 8,
**caractérisé en ce que**
le support synthétisé subséquemment est synthétisé avec des récepteurs prolongés vis-à-vis du premier support.

10. Procédé selon l'une des revendications 1 à 8,
**caractérisé en ce que**
le support synthétisé subséquemment est synthétisé avec des récepteurs modifiés par rapport au premier support.

11. Procédé selon la revendication 10,
**caractérisé en ce que**
la modification des récepteurs comporte une modification de la séquence et/ou une exclusion des récepteurs négatifs du cycle précédent.

12. Procédé selon l'une des revendications 1 à 11,
**caractérisé en ce que**
l'on utilise un support plan.

13. Procédé selon l'une des revendications 1 à 12,
**caractérisé en ce que**
l'on utilise un support ayant une pluralité de canaux.

14. Procédé selon l'une des revendications 1 à 13,
**caractérisé en ce que**
lesdits plusieurs supports sont synthétisés et analysés dans différents dispositifs de détection, qui sont reliés entre eux par des moyens techniques d'information, mais peuvent être séparés les uns des autres du point de vue spatial.

15. Utilisation du procédé selon l'une des revendications 1 à 14 pour la détermination d'un analyte dans un échantillon.

16. Utilisation selon la revendication 15 pour le séquençage d'acides nucléiques.

17. Utilisation selon la revendication 16 pour le nouveau séquençage et/ou le reséquençage de matériau génétique complexe, tel que par exemple un génome individuel ou des acides nucléiques synthétiques.

18. Utilisation selon la revendication 15 pour l'obtention d'informations diagnostiques pour le traitement individuel d'un patient, comme par exemple l'action individuelle d'agents pharmaceutiques.

19. Utilisation selon la revendication 15 pour l'analyse d'activité de substances pharmacologiques.

20. Utilisation selon la revendication 15 pour la constitution et l'analyse de banques de substances.

21. Utilisation selon la revendication 15 pour la comparaison d'individus dans une population.
